# EUROPEAN PATENT SPECIFICATION

(11) **EP 3 440 111 B1**
(45) Date of publication and mention of the grant of the patent: **20.12.2023**
(21) Application number: 17725339.0
(22) Date of filing: 05.04.2017
(51) Int. Cl.: C07K 16/28, C07K 16/30, C07K 7/00, A61K 39/395

(54) **ANTI-VEGFR-1 ANTIBODIES AND USES THEREOF**
ANTI-VEGFR-1-ANTIKÖRPER UND VERWENDUNGEN DAVON
ANTICORPS ANTI-VEGFR-1 ET LEURS UTILISATIONS

(30) Priority: 05.04.2016 IT UA20162326
(43) Date of publication of application: 13.02.2019
(73) Proprietor: Fondazione Luigi Maria Monti, 00167 Rome (IT); Università degli Studi di Roma " Tor Vergata", 00173 Rome (IT)
(72) Inventor: LACAL SANJUAN, Pedro Miguel, 00133 Rome (IT); D'ATRI, Stefania, 00179 Rome (IT); MOREA, Veronica, 00198 Rome (IT); TENTORI, Lucio, 00141 Rome (IT); GRAZIANI, Grazia, 00133 Rome (IT); RUFFINI, Federica, 00169 Rome (IT); FAILLA, Maria Cristina, 00199 Rome (IT)
(74) Representative: Ghirardi, Valeria
(86) International application number: PCT/IB2017/000379
(87) International publication number: WO 2017/175054

(56) References cited:
- WO-A2-2006/055809
- WO-A2-2008/015720
- Anonymous: "Ibridoma murino secernente l'anticorpo monoclonale D16F7.9 diretto verso il vascular endothelial growth factor receptor (VEGFR)-1 | BioTTasa DB", , 1 March 2014 (2014-03-01), XP055323633, Retrieved from the Internet: URL:http://biottasadb.cbm.fvg.it/tecnologi e/ibridoma-murino-secernente-l'anticorpo-m onoclonale-d16f79-diretto-verso-il-vascula r [retrieved on 2016-11-28]
- GRAZIA GRAZIANI ET AL: "Antitumor activity of a novel anti-vascular endothelial growth factor receptor-1 monoclonal antibody that does not interfere with ligand binding", ONCOTARGET, vol. 7, no. 45, 19 September 2016 (2016-09-19), pages 72868-72885, XP055323629, DOI: 10.18632/oncotarget.12108
- Y. WU ET AL: "Anti-Vascular Endothelial Growth Factor Receptor-1 Antagonist Antibody as a Therapeutic Agent for Cancer", CLINICAL CANCER RESEARCH, vol. 12, no. 21, 1 November 2006 (2006-11-01), pages 6573-6584, XP055120093, ISSN: 1078-0432, DOI: 10.1158/1078-0432.CCR-06-0831
- WENBO LIU ET AL: "Tumor-derived vascular endothelial growth factor (VEGF)-A facilitates tumor metastasis through the VEGF-VEGFR1 signaling pathway", INTERNATIONAL JOURNAL OF ONCOLOGY, vol. 39, 22 July 2011 (2011-07-22), pages 1213-1220, XP055324024, GR ISSN: 1019-6439, DOI: 10.3892/ijo.2011.1138
- MARIA GRAZIA ATZORI ET AL: "The anti-vascular endothelial growth factor receptor-1 monoclonal antibody D16F7 inhibits invasiveness of human glioblastoma and glioblastoma stem cells", JOURNAL OF EXPERIMENTAL & CLINICAL CANCER RESEARCH, vol. 36, no. 1, 10 August 2017 (2017-08-10), XP055610091, DOI: 10.1186/s13046-017-0577-2
- MARIA GRAZIA ATZORI ET AL: "The Anti-Vascular Endothelial Growth Factor Receptor-1 Monoclonal Antibody D16F7 Inhibits Glioma Growth and Angiogenesis In Vivo", JOURNAL OF PHARMACOLOGY AND EXPERIMENTAL THERAPEUTICS, vol. 364, no. 1, 12 October 2017 (2017-10-12), pages 77-86, XP055610093, US ISSN: 0022-3565, DOI: 10.1124/jpet.117.244434
- KLIMKA A ET AL: "HUMAN ANTI-CD30 RECOMBINANT ANTIBODIES BY GUIDED PHAGE ANTIBODY SELECTION USING CELL PANNING", BRITISH JOURNAL OF CANCER, NATURE PUBLISHING GROUP, GB, vol. 83, no. 2, 1 July 2000 (2000-07-01), pages 252-260, XP001021661, ISSN: 0007-0920, DOI: 10.1054/BJOC.2000.1226
- SHANA M. BARBAS ET AL: "Recognition of DNA by Synthetic Antibodies", JOURNAL OF THE AMERICAN CHEMICAL SOCIETY, vol. 116, no. 5, 1 March 1994 (1994-03-01) , pages 2161-2162, XP055139934, ISSN: 0002-7863, DOI: 10.1021/ja00084a073
- BARBAS S M ET AL: "HUMAN AUTOANTIBODY RECOGNITION OF DNA", PROCEEDINGS OF THE NATIONAL ACADEMY OF SCIENCES, NATIONAL ACADEMY OF SCIENCES, vol. 92, 1 March 1995 (1995-03-01), pages 2529-2533, XP002927212, ISSN: 0027-8424, DOI: 10.1073/PNAS.92.7.2529
- Hj Ditzel ET AL: "Determinants of polyreactivity in a large panel of recombinant human antibodies from HIV-1 infection", The Journal of Immunology, 15 July 1996 (1996-07-15), pages 739-749, XP055569965, United States Retrieved from the Internet: URL:http://www.jimmunol.org/content/jimmun ol/157/2/739.full.pdf
- BEIBOER S H W ET AL: "Guided selection of a pan carcinoma specific antibody reveals similar binding characteristics yet structural divergence between the original murine antibody and its human equivalent", JOURNAL OF MOLECULAR BIOLOGY, ACADEMIC PRESS, UNITED KINGDOM, vol. 296, no. 3, 25 February 2000 (2000-02-25), pages 833-849, XP004461580, ISSN: 0022-2836, DOI: 10.1006/JMBI.2000.3512
- XU JOHN L ET AL: "Diversity in the CDR3 region of VH is sufficient for most antibody specificities", IMMUNITY, CELL PRESS, US, vol. 13, no. 1, 1 July 2000 (2000-07-01), pages 37-45, XP009147376, ISSN: 1074-7613, DOI: 10.1016/S1074-7613(00)00006-6 [retrieved on 2000-09-05]
- DANIÈLE GILBERT ET AL: "A complementarity-determining region peptide of an anti-desmosome autoantibody may interact with the desmosomal plaque through molecular mimicry with a cytoplasmic desmoglein 1 sequence", EUROPEAN JOURNAL OF IMMUNOLOGY, vol. 27, no. 5, 1 May 1997 (1997-05-01), pages 1055-1060, XP055139938, ISSN: 0014-2980, DOI: 10.1002/eji.1830270503
- Alan Berezov ET AL: "S C I E N T I F I C Biacore analysis of rationally designed anti-HER2 exocyclic mimetics of antibodies Reprinted from BIAjournal 8 (1) 2001", , 1 January 2001 (2001-01-01), XP055361257, Retrieved from the Internet: URL:http://www.protein.iastate.edu/seminar s/BIACore/ScientificReviews/ScientificRevi ew8.pdf [retrieved on 2017-04-03]
- MONNET C ET AL: "Synthetic peptides derived from the variable regions of an anti-CD4 monoclonal antibody bind to CD4 and inhibit HIV-1 promoter activation in virus-infected cells", JOURNAL OF BIOLOGICAL CHEMISTRY, AMERICAN SOCIETY FOR BIOCHEMISTRY AND MOLECULAR BIOLOGY, US, vol. 274, no. 6, 5 February 1999 (1999-02-05), pages 3789-3796, XP002283342, ISSN: 0021-9258, DOI: 10.1074/JBC.274.6.3789
- LACAL P M ET AL: "Inhibition of endothelial cell migration and angiogenesis by a vascular endothelial growth factor receptor-1 derived peptide", EUROPEAN JOURNAL OF CANCER, ELSEVIER, AMSTERDAM, NL, vol. 44, no. 13, 1 September 2008 (2008-09-01), pages 1914-1921, XP023979229, ISSN: 0959-8049, DOI: 10.1016/J.EJCA.2008.06.032 [retrieved on 2008-08-03]
- JIN HONG KIM ET AL: "Enhanced humanization and affinity maturation of neutralizing anti-hepatitis B virus preS1 antibody based on antigen-antibody complex structure", FEBS LETTERS, ELSEVIER, AMSTERDAM, NL, vol. 589, no. 2, 4 December 2014 (2014-12-04), pages 193-200, XP071254880, ISSN: 0014-5793, DOI: 10.1016/J.FEBSLET.2014.11.046
- MAGDELAINE-BEUZELIN ET AL: "Structure-function relationships of the variable domains of monoclonal antibodies approved for cancer treatment", CRITICAL REVIEWS IN ONCOLOGY/HEMATOLOGY, ELSEVIER, AMSTERDAM, NL, vol. 64, no. 3, 14 November 2007 (2007-11-14), pages 210-225, XP022344458, ISSN: 1040-8428, DOI: 10.1016/J.CRITREVONC.2007.04.011
- JAMES R. APGAR ET AL: "Beyond CDR-grafting: Structure-guided humanization of framework and CDR regions of an anti-myostatin antibody", MABS, vol. 8, no. 7, 13 September 2016 (2016-09-13), pages 1302-1318, XP055416065, US ISSN: 1942-0862, DOI: 10.1080/19420862.2016.1215786

## Description

### TECHNICAL FIELD OF THE INVENTION

The present invention relates to anti-VEGFR (Vascular Endothelial Growth Factor Receptor) antibodies, in particular anti-VEGFR-1 antibodies in the treatment of tumours.

### PRIOR ART

Melanoma is a highly aggressive tumour characterized by an early metastatic spread and poor response to chemotherapy and radiotherapy. Despite numerous experimental clinical protocols and the recent introduction of new molecular target drugs in the therapy of melanoma (i.e., the immunostimulant monoclonal antibodies ipilimumab, pembrolizumab, nivolumab, and the kinase inhibitors vemurafenib, dabrafenib, trametinib), the median overall survival for the metastatic disease is still a few months (Spagnolo F, 2016). It is therefore all the more important to gain more in-depth knowledge on the biology of melanoma in order to identify new therapeutic targets and molecular markers useful for the diagnosis and the assessment of prognosis, and for new therapeutic approaches.

Several experimental evidences indicate that different angiogenic factors and their receptors are able to support autocrine proliferative and metastatic loops in tumours from various histological nature, including melanoma. Among the angiogenic factors, those belonging to the vascular endothelial growth factor family, and in particular VEGF-A and placental growth factor (PIGF), play a key role in these processes. VEGF-A interacts with two tyrosine kinase membrane receptors (Lohela, 2009): VEGFR-1 [NCBI = P17948] (involved in the activation of cell migration and in the modulation of the signal transduced by VEGFR-2) and VEGFR-2 [NCBI = P35968] (mainly responsible for the signal transduction mediating the proliferative and permeabilizing effects of VEGF-A). PlGF, however, is able to bind only VEGFR-1. In addition to as a membrane receptor (mVEGFR-1), VEGFR-1 is also produced as a soluble protein (sVEGFR-1), comprising the entire extracellular amino acid sequence of the membrane receptor and a specific sequence of 31 amino acids at its C-terminal end, below underlined (SEQ ID No. 1, Kendall, 1996) :

1 MVSYWDTGVL LCALLSCLLL TGSSSGSKLK DPELSLKGTQ HIMQAGQTLH LQCRGEAAHK
61 WSLPEMVSKE SERLSITKSA CGRNGKQFCS TLTLNTAQAN HTGFYSCKYL AVPTSKKKET
121 ESAIYIFISD TGRPFVEMYS EIPEIIHMTE GRELVIPCRV TSPNITVTLK KFPLDTLIPD
181 GKRIIWDSRK GFIISNATYK EIGLLTCEAT VNGHLYKTNY LTHRQTNTII DVQISTPRPV
241 KLLRGHTLVL NCTATTPLNT RVQMTWSYPD EKNKRASVRR RIDQSNSHAN IFYSVLTIDK
301 MQNKDKGLYT CRVRSGPSFK SVNTSVHIYD KAFITVKHRK QQVLETVAGK RSYRLSMKVK
361 AFPSPEVVWL KDGLPATEKS ARYLTRGYSL IIKDVTEEDA GNYTILLSIK QSNVFKNLTA
421 TLIVNVKPQI YEKAVSSFPD PALYPLGSRQ ILTCTAYGIP QPTIKWFWHP CNHNHSEARC
481 DFCSNNEESF ILDADSNMGN RIESITQRMA IIEGKNKMAS TLVVADSRIS GIYICIASNK

541 VGTVGRNISF YITDVPNGFH VNLEKMPTEG EDLKLSCTVN KFLYRDVTWI LLRTVNNRTM

601 HYSISKQKMA ITKEHSITLN LTIMNVSLQD SGTYACRARN VYTGEEILQK KEITIRGEHC

661 NKKAVFSRIS KFKSTRNDCT TQSNVKH

sVEGFR-1 acts both as a negative regulator of the activity of VEGF-A and PlGF (by binding these factors and preventing their interaction with membrane receptors) and as a molecule involved in cell adhesion and migration. sVEGFR-1, in fact, is localized in the extracellular matrix and is able, by interacting with the integrin α5β1, to promote cell adhesion and migration (Orecchia, 2003).

Recently, the authors of the present invention have evaluated the expression of the soluble and membrane form of VEGFR-1 by real-time RT-PCR in a panel of 36 melanoma cell lines, derived from primary and metastatic lesions, and in 11 primary cultures of human melanocytes (Ruffini, 2011). The results obtained demonstrate that mVEGFR-1 and sVEGFR-1 are not present in normal melanocytes, while the two receptor isoforms are highly expressed in the majority (~90%) of the melanoma cell lines derived from primary lesions. In addition, the expression of mVEGFR-1 and sVEGFR-1 is found in 70% and 50%, respectively, of the cell lines derived from metastasis. In cutaneous metastases in particular the sVEGFR-1/mVEGFR-1 ratio is significantly reduced compared to the lines derived from primary lesions. These data suggest that, in the initial phase of melanoma development, sVEGFR-1, by acting mainly as an extracellular matrix component, could promote the adhesion and migration of endothelial and tumour cells, while during progression such soluble receptor could act predominantly by limiting the amount of free VEGF-A capable of stimulating the membrane receptors, controlling the invasiveness of tumour cells. A reduced sVEGFR-1/VEGF-A ratio could then be a poor prognosis indicator in melanoma.

The involvement of VEGFs and VEGFRs both in angiogenesis and in proliferation and invasive ability of cancer cells, makes these factors important targets for anti-cancer therapies. For example, WO03/006059 relates to methods for inhibiting cancer cells through administration of an antagonist, such as an antibody, which inhibits the VEGF/VEGFR-1 autocrine loop of tumour cells. WO2011159704 relates to monoclonal antibodies that specifically bind VEGF. These antibodies block the binding of VEGF to its receptors (for example, VEGFR1 and/or VEGFR2) and prevent the phosphorylation of VEGFR-2 by VEGF. The patent application also relates to methods of use of anti-VEGF monoclonal antibodies for the treatment of pathologies, including cancer. WO2008/015720 refers to antibodies binding to a peptide in VEGFR-1 domain II. Currently, the clinical use of bevacizumab, a humanized anti-VEGF-A monoclonal antibody, is approved in combination with chemotherapy for the treatment of colorectal cancer and (only by EMA) of metastatic breast cancer, advanced, recurrent or metastatic lung non-small cell cancer, ovarian cancer and advanced or recurrent cervical cancer; in combination with interferon alfa-2a for the treatment of advanced or metastatic kidney carcinoma, and as monotherapy (only by FDA) for recurrent glioblastoma multiforme (Arjaans, 2016). In addition, bevacizumab is under clinical trial for the treatment of other cancers, including melanoma (Karimkhani, 2014). Tyrosine kinase inhibitors non-selective for the various VEGFRs are also used in oncology, and an anti-VEGFR-2 monoclonal antibody (ramucirumab) was recently approved for gastric cancer and advanced gastro-esophageal junction cancer (Arjaans, 2016). Also different anti-VEGFR-3 or anti-VEGFR-2 antibodies are known in the treatment of different pathologies. WO2012033696 relates for example to anti-VEGFR-3 monoclonal antibodies, pharmaceutical compositions containing said antibodies, and the use of antibodies in the treatment of diseases including, for example, ovarian cancer, breast cancer, kidney cell carcinoma, pancreatic cancer, lung cancer and colon cancer. The possible role of VEGFR-1 as a specific target for anticancer therapies, however, remains largely unexplored. In this respect, it is important to emphasize that VEGFR-1 and PIGF, in contrast to VEGF-A and VEGFR-2, don't have an important role in the physiological angiogenesis processes that occur in the adult, but they are mainly associated with the angiogenesis that characterizes a variety of pathological processes (e.g., tumours, exudative age-related retinal macular degeneration, diabetic retinopathy, etc.) (Fischer, 2008; Dewerchin, 2014). The strategy used in the production of monoclonal antibodies specific for VEGFR-1, (IMC-18F1 antibody, CA2590671 A1), consisted of the selection of reagents that inhibit receptor binding of its major ligands: VEGF-A and PIGF. WO2014150314 relates to methods of use of anti-VEGFR-1 antibodies for the treatment of chronic kidney diseases. These antibodies neutralize the binding of VEGF-A and PIGF to VEGFR-1. WO2006/055809 for example refers to monoclonal antibodies specific for VEGFR-1 for the treatment of disorders related to angiogenesis, including tumours. These antibodies prevent the binding of VEGFR-1 to ¹²⁵I-VEGF and inhibit the phosphorylation of VEGFR-1 triggered by VEGF or PlGF, by inhibiting the binding to the receptor of VEGF-A and PIGF. However, the known anti-VEGFR-1 antibodies, by preventing the binding of VEGF-A also to the soluble form of the receptor, even abolish the inhibitory function on the metastatic process of the same. Recently, the authors have demonstrated the anti-angiogenic and anti-tumour properties of a peptide (peptide A4) that comprises a sequence of the second extracellular domain of VEGFR-1 receptor, suggesting that the sequence of the receptor comprised in this peptide may have a key role in the functionality of VEGFR-1 (Lacal, 2008). The peptide, unfortunately, has a reduced stability under physiological conditions and, therefore, is likely to have a poor therapeutic efficacy. Therefore, there is a need to provide an antibody capable of specifically binding VEGFR-1 and inhibiting its activation as a membrane receptor, without interfering with the binding of VEGF-A or PIGF to the soluble form of the receptor and with its beneficial anti-proliferative activities in general, and, in particular, with its anticancer and antimetastatic activities.

### DISCLOSURE OF INVENTION

The authors of the present invention have produced a monoclonal antibody, called D16F7, obtained by immunizing BALB/c mice with a peptide, called A4, derived from the sequence of VEGFR-1, and modified. The hybridoma secreting this antibody was selected based on the binding specificity to VEGFR-1 by ELISA and Western blotting assays. D16F7 is an IgG1 having the unexpected properties of inhibiting the migration in response to VEGF-A and PlGF of human endothelial, myeloid and tumour (melanoma and glioblastoma) cells, and the *in vitro* invasiveness of the latter, without interfering with the binding of VEGF-A or PIGF to the receptor. In addition, the antibody negatively modulates the homodimerization of VEGFR-1 and its phosphorylation in response to VEGF-A or PIGF, has a marked anti-angiogenic and antitumor activity in *in vivo* assays, and is well tolerated after intraperitoneal injection of up to 40 mg/kg in a mouse model. The use of the monoclonal antibody D16F7 has several advantageous properties from a therapeutic point of view. The high stability in the systemic circulation and the long half-life of the IgG-type antibodies in general, and in particular of D16F7, solves the problem of poor stability of small peptides when used in therapeutic compositions and, therefore, increases therapeutic efficacy compared to them. The monoclonal antibody D16F7 does not prevent the formation of the binding of VEGF-A and/or PlGF to VEGFR-1, but inhibits the subsequent activation of the membrane receptor, inhibiting the transmission of the signal inside the cell and angiogenesis. Also, it does not prevent the binding of VEGF-A to the receptor soluble form, allowing the latter to continue to perform its inhibitory function on the metastatic process. Therapeutic strategies that target VEGF-A or VEGFR-2 give rise to systemic toxicity problems, due to the inhibition of physiological angiogenesis, resulting in bleeding, hypertension, delayed wound healing, etc. (Norden, 2009; Higa, 2009). The anti-angiogenic therapy with the antibody of the invention, which specifically targets VEGFR-1 involved in the angiogenesis associated with cancer and not in the physiological angiogenesis, shows less systemic toxicity compared to therapies directed towards VEGF-A and/or VEGFR-2 and, if it is used in combination with them, determines an amplification of the clinical response, without having additive toxicity. In addition, the few molecules developed until today with the aim of interfering solely with the VEGFR-1 activity have been designed to inhibit the binding of VEGF-A and PlGF to this receptor. Since VEGF-A has an affinity for VEGFR-1 higher of about 10 times compared to that for VEGFR-2, an antibody that does not compete with the binding of VEGF-A to VEGFR-1 requires a lower concentration in order to obtain an antitumor effect than it would require an antibody that inhibits the activity of the receptor in a non-competitive way. The monoclonal antibody D16F7, or synthetic or recombinant fragments or derivatives thereof, that maintain the binding specificity and functionality, therefore allows a reduction in treatment costs. The expression of VEGFR-1 by the tumour, and the inhibitory effect on the migration and invasion of tumour cells by D16F7 indicate that this antibody can exert a control over the process of metastatic spreading, even independently from its anti-angiogenic activity. The monoclonal antibody D16F7 may be humanized according to techniques known to the skilled person, such as for example those described by Villani and collaborators (Villani ME, 2008), for use in therapy, in all clinical processes in which angiogenesis is an important component in the development and/or progression of the disease, such as, for example, the development of tumours, diabetic retinopathy, and neovascularization in exudative age-related macular degeneration. For research, the availability of a monoclonal antibody able to specifically interfere with the activation of VEGFR-1 is a useful tool for studying the biological role of VEGFR-1 in angiogenesis, proliferation and tumour metastasis processes. Results similar to those described in the present application were also obtained by the inventors in human glioblastoma cells (cell lines derived from patients and stem cell cultures) and in nude mice grafted with glioblastoma cells expressing high levels of VEGFR-1. It is an object of the present invention an antibody or a synthetic or recombinant fragment thereof able to recognize and bind to an epitope comprised in the sequence from aa. 149 to aa. 161 of SEQ ID No. 1 of VEGFR-1, and not to prevent the binding of VEGF-A or PlGF to VEGFR-1, comprising:
- a complementarity determining region 3 of the heavy chain (CDRH3) having the sequence YLGDY (aa. 118-122 of SEQ. ID No. 23), and
- a complementarity determining region 2 of the heavy chain (CDRH2) having the sequence EIYPRNGNTNYDEKFKG (aa. 69-85 of SEQ. ID No. 23), and
- a complementarity determining region 1 of the heavy chain (CDRH1) having the sequence GYIFTNYWMH (aa. 45-54 of SEQ. ID No. 23), and
- a complementarity determining region 3 of the light chain (CDRL3) having the sequence FQGSHVPYT (aa. 113-121 of SEQ. ID No. 25), and
- a complementarity determining region 2 of the light chain (CDRL2) having the sequence KVSNRFS (aa. 74-80 of SEQ. ID No. 25), and
- a complementarity determining region 1 of the light chain (CDRL1) having the sequence RSSQSIVHSNGNTYLE (aa. 43-58 of SEQ. ID No. 25).

According to a preferred embodiment, the antibody or a synthetic or recombinant fragment thereof is able to recognize and bind to an epitope comprised in the sequence from aa. 156 to aa. 161 of SEQ ID No. 1 of VEGFR-1, and not to prevent the binding of VEGF-A or PlGF to VEGFR-1. Preferably, the antibody or a synthetic or recombinant fragment thereof is obtained using as antigen the peptide of sequence TEGRELVIPARVT (SEQ ID No. 2).

In a preferred embodiment, the antibody or a synthetic or recombinant fragment thereof comprises:
- the variable region of the heavy chain comprising essentially the amino acid sequence: QVQLQQPGSELVRPGASVKLSCKASGYIFTNYWMHWVKQRPGQGLEWIGEIYPRNGNT NYDEKFKGKATLTIDTSSSTAYMQLSSLTSEDSAVYYCATYLGDYWGQGTTLTVSS (aa. 20-133 of SEQ ID No. 23) and/or
- the variable region of the light chain comprising essentially the amino acid sequence: DVLMTQTPLSLPVSLGDQASISCRSSQSIVHSNGNTYLEWFLQKPGQSPKLLIYKVSNRFS GIPDRFSGSGSGTDFTLKISRVEAEDLGVYFCFQGSHVPYTFGGGTKLEIK (aa. 20-131 of SEQ ID No. 25).

The antibody or a synthetic or recombinant fragment thereof of the invention can be a monoclonal, chimeric, humanized or deimmunized antibody.

Preferably, the antibody is selected from the group consisting of an intact immunoglobulin (or antibody), a Fv, a scFv (single chain Fv fragment), a Fab, a F(ab')₂, an antibody-like domain, an antibody-mimetic domain, a single antibody domain, a multimeric antibody, a peptide or a proteolytic fragment containing the epitope binding region. The term "antibody" in the present invention is used in the most general sense, and encompasses various antibodies and antibody mimetic structures, including, but not limited to, monoclonal antibodies, polyclonal antibodies, multispecific antibodies (e.g., bispecific antibodies), human antibodies, humanized antibodies, deimmunized antibodies, chimeric antibodies, nanobodies, antibody derivatives, antibody fragments and other binding domains, provided that they show desired binding activity for the antigen. An "antibody fragment" refers to a molecule other than an intact antibody that comprises a portion of an intact antibody that binds the antigen to which the intact antibody binds. Examples of antibody fragments include, but are not limited to, Fv, Fab, Fab', Fab'-SH, F(ab')2; diabody; linear antibodies; single-chain antibody molecules (e.g. scFv); and multispecific antibodies consisting of antibody fragments. Fv of VH and VL are also called "nanobodies". The term "mimetic antibody" refers to those organic compounds or binding domains that are not antibody derivatives but that can specifically bind to an antigen, in the same way of the antibodies. The term "chimeric" antibody refers to an antibody in which a portion of the heavy and/or light chain is derived from one specific source or species, while the remainder of the heavy and/or light chain is derived from a different source or species.

The terms "full-length antibody," "intact antibody" and "whole antibody" are used herein interchangeably to refer to an antibody having a structure substantially similar to a native antibody structure or having heavy chains that contain a Fc region as defined herein. A "human antibody" is one that possesses an amino acid sequence which corresponds to that of an antibody produced by a human being or a human cell or derived from a non-human source that uses repertoires of human antibodies or other sequences encoding human antibodies. This definition of a human antibody specifically excludes a humanized antibody comprising non-human antigen-binding residues. In humans, the antibody isotypes are IgA, IgD, IgE, IgG and IgM. An antibody "humanized" refers to a chimeric antibody comprising amino acid residues from non-human hypervariable regions (HVR) and amino acid residues from the remaining human regions (FR: Framework Regions). In certain embodiments, a humanized antibody will comprise substantially at least an entire variable domain, and typically two, in which all or substantially all of the HVRs (for example, CDRs) correspond to those of a non human antibody, and all or substantially all of the FRs correspond to those of a human antibody. A humanized antibody optionally may comprise at least a portion of an antibody constant region derived from a human antibody. A "humanized form" of an antibody, for example, a non-human antibody, refers to an antibody subjected to humanization. An antibody "deimmunized" is an antibody with reduced immunogenicity based on the destruction of HLA binding, a basic requirement for the stimulation of T cells. The term "monoclonal antibody" as used herein refers to an antibody obtained from a population of substantially homogeneous antibodies, i.e., the individual antibodies comprising the population are identical and/or bind the same epitope, except for possible antibodies variants, for example, containing naturally occurring mutations or that are generated during the production of a monoclonal antibody preparation, such variants generally being present in lower amounts. In contrast to polyclonal antibody preparations, which typically include different antibodies directed against different determinants (epitopes), each monoclonal antibody of a monoclonal antibody preparation is directed against a single determinant on an antigen. Therefore, the modifier "monoclonal" indicates that the character of the antibody is obtained from a substantially homogeneous population of antibodies, and should not be interpreted as the need to produce the antibody by any particular method. For example, the monoclonal antibodies to be used according to the present invention can be produced by a variety of techniques, including, but not limited to, the hybridoma method, methods based on recombinant DNA, phage display methods, and methods that use transgenic animals containing all or part of human immunoglobulin loci. In the context of the present invention, an "antibody that binds to VEGFR-1, and does not prevent the binding of VEGF-A or PlGF to VEGFR-1" includes modifications of the antibody according to the present invention able to maintain the specificity mentioned above. These changes include, for example, the conjugation to effector molecules such as chemotherapeutic or cytotoxic agents, and/or detectable reporter portions.

The term "variable region" or "variable domain" refers to the domain of a heavy or light chain of antibody that is involved in the binding of the antibody to the antigen. The variable domains (or regions) of the heavy and light chain (VH and VL, respectively) of a native antibody generally have similar structures, each domain comprising four framework conserved regions (FR) and three hypervariable regions (HVR, see, for example, Kindt et al. Kuby Immunology, 6th ed., W.H. Freeman and Co., page 91, 2007). A single VH or VL domain can be sufficient to confer antigen binding specificity. Moreover, it is possible to isolate antibodies that bind to a specific antigen using a VH or VL domain from an antibody that binds the antigen to screen a library of complementary VL or VH domains, respectively (see, for example, Portolano et al., J. Immunol. 150:880- 887, 1993; Clarkson et al., Nature 352:624-628, 1991).

In another aspect, the antibody or derivatives thereof comprise a variable domain sequence of the heavy chain (VH) and/or a variable domain sequence of the light chain (VL) having at least 80%, 85%, 90%, 91%, 92%, 93%, 94%, 95%, 96%, 97%, 98%, 99%, or 100% sequence identity to each of the CDRs comprised in the amino acid sequences of SEQ ID No: 23 or SEQ ID No: 25, respectively, as defined above.

In certain embodiments, the VH sequence and/or the VL sequence having at least 80%, 85%, 90%, 91%, 92%, 93%, 94%, 95%, 96%, 97%, 98%, or 99% identity to said SEQ ID No. 23 or SEQ ID No: 25, respectively, contains substitutions (e.g., conservative substitutions), insertions, or deletions as compared to the reference sequence, however, the anti-VEGFR-1 antibody comprising this sequence maintains the ability to bind to the epitope. The antibody-like domain comprises binding proteins structurally related to antibodies, such as T cell receptors. The antibodies of the present invention also include functional equivalents that include polypeptides with amino acid sequences substantially identical to the amino acid sequence of the variable or hypervariable regions of the antibodies of the present invention. Sequences of the invention comprise amino acid sequences with at least 70, preferably at least 80%, and more preferably at least 90% identity or homology to the sequences described. "The percent (%) amino acid sequence identity" with respect to a reference polypeptide sequence is defined as the percentage of amino acid residues in a candidate sequence that are identical to the amino acid residues in the reference polypeptide sequence, after aligning the sequences and introducing gaps, if necessary, to achieve maximum percent sequence identity, and not considering any conservative substitutions as part of the sequence identity. The alignment in order to determine the percent of amino acid sequence identity can be achieved in various ways that are within the skill in the art, for instance, using publicly available computer software such as BLAST, BLAST-2, ALIGN or Megalign software (DNASTAR). Those skilled in the art can determine appropriate parameters for aligning sequences, including any algorithms needed to achieve maximal alignment over the full-length of the sequences being compared. The antibody or synthetic or recombinant antigen binding fragments thereof preferably have a dissociation constant (Kd) of <100 nM, <10 nM, <1 nM, <0.1 nM, <0.01 nM, or <0.001 nM or less, e.g. from 10 ⁻⁸ M to 10 ⁻¹³ M, e.g. , from 10 ⁻⁹ M to 10 ⁻¹³ M. Recombinant and/or biotechnological derivatives as well as fragments of the antibodies described above are included within the invention, provided that the binding activity of the antibodies and their functional specificity is maintained. The antibody or a synthetic or recombinant fragment thereof of the invention is used as a medicament, preferably for use in the treatment and/or prevention of pathologies related to angiogenesis such as for use in inhibiting angiogenesis or for use in therapy to obtain a reduction of tumour growth in a mammal. The diseases that can be treated with the antibody of the invention are preferably tumour diseases, such as, for example, melanoma, glioblastoma, colorectal carcinoma, breast carcinoma, kidney carcinoma, ovary carcinoma, cervical carcinoma, non-small cell lung carcinoma and/or metastasis, retinopathies as diabetic retinopathy, neovascularization in age-related exudative macular degeneration, macular oedema induced by occlusion of retina artery, choroid neovascularization caused by pathologic myopia.

In the context of the present invention, in addition to cancers, the angiogenesis-related diseases include pathologies characterized by an excessive angiogenesis, involving, for example, inflammation and/or vascularization, and include, for example, rheumatoid arthritis, neovascular glaucoma, macular degeneration, psoriasis, proliferative retinopathy, diabetes, multiple sclerosis, allergic inflammation, osteoporosis, septic shock.

In the context of the present invention, the "cancer" or "tumour" includes primary and metastatic tumours, as well as refractory tumours, tumours expressing VEGFR-1, solid or non-solid tumours. Examples of solid tumours are: breast cancer, carcinoma, lung cancer, colorectal carcinoma, pancreatic carcinoma, glioma, lymphoma, prostate cancer, thyroid cancer, ovarian cancer, liver cancer, neuroblastoma, melanoma. Examples of non-solid tumours are: leukemia, multiple myeloma and lymphoma. It is a further aspect of the invention a nucleic acid molecule encoding the antibody or a synthetic or recombinant fragment thereof of the invention, preferably comprising a nucleotide sequence essentially consisting of : 
CAGGTCCAACTGCAGCAACCTGGGTCTGAACTGGTGAGGCCTGGAGCTTCAGTGAA
GCTGTCCTGCAAGGCTTCTGGCTACATATTCACCAACTACTGGATGCACTGGGTGAA
GCAGAGGCCTGGACAAGGCCTTGAGTGGATTGGAGAAATTTATCCTCGTAATGGTA
ATACTAACTACGATGAGAAGTTCAAGGGCAAGGCCACACTGA
CTATAGACACATCCTCCAGCACAGCCTACATGCAGCTCAGCAGCCTGACATCTGAGG
ACTCTGCGGTCTATTACTGTGCCACCTACCTAGGGGACTACTGGGGCCAAGGCACCA
CTCTCACAGTCTCCTCA (nt. 58-399 of SEQ ID No. 22)
 and/or

GATGTTTTGATGACCCAAACTCCACTCTCCCTGCCTGTCAGCCTTGGAGATCAAGCC
TCCATCTCTTGCAGATCTAGTCAGAGTATTGTTCATAGTAATGGAAACACCTATTTA
GAATGGTTCCTGCAGAAACCAGGCCAGTCTCCAAAGCTCCTGATCTACAAAGTTTCC
AACCGATTTTCTGGGATCCCAGACAGGTTCAGTGGCAGTGGATCAGGGACAGATTTC
ACACTCAAGATCAGTAGAGTGGAGGCTGAGGATCTGGGAGTTTATTTCTGCTTTCAA
GGTTCACATGTTCCGTACACGTTCGGAGGGGGGACCAAGCTGGAAATAAAA (nt. 58-
393 of SEQ ID No. 24).

A further aspect of the present invention is a nucleic acid encoding the antibody or antigen binding fragments thereof of the invention, or consisting of a correspondent degenerated sequence.

It is within the scope of the invention an expression vector encoding the antibody or a synthetic or recombinant fragment of the invention, preferably comprising the nucleic acid as defined above. It is within the scope of the invention a host cell comprising the nucleic acid as defined above, or the vector as defined above.

The terms "host cell", "host cell line", and "host cell culture" are used interchangeably and refer to cells into which an exogenous nucleic acid has been introduced, including the progeny of such cells. The host cells include "transformants" and "transformed cells," which include the transformed primary cell and the progeny derived therefrom, without taking into account the number of steps. The progeny may be not completely identical in nucleic acid content to a parent cell, but may contain mutations. In the present invention mutant progenies are included, which have the same function or biological activity as that for which they have been screened or selected in the originally transformed cell. The nucleic acids of the invention can be used to transform a suitable mammalian host cell. Mammalian cells available as expression hosts are well known and include, for example, CHO and BHK cells. Prokaryotic hosts include, for example, E. coli, Pseudomonas, Bacillus, etc. Antibodies of the invention can be fused to additional amino acid residues, such as tags that facilitate their isolation. The term "vector", as used in the present invention refers to a nucleic acid molecule capable of propagating another nucleic acid to which it is linked. The term includes the vector as a self-replicating nucleic acid structure as well as the vector incorporated into the genome of a host cell in which it was introduced. Certain vectors are capable of directing the expression of nucleic acids to which they are operably linked. In the present such vectors are referred to as "expression vectors." Any suitable expression vector can be used, for example prokaryotic cloning vectors such as plasmids from E. coli, such as colE1, pCR1, pBR322, pMB9, pUC. Expression vectors suitable for expression in mammalian cells include derivatives of SV-40, adenovirus, retrovirus-derived DNA sequences. The expression vectors useful in the present invention contain at least one expression control sequence that is operatively linked to the sequence or fragment of DNA that must be expressed. It is a further object of the invention a method of treatment and/or prevention of angiogenesis-related diseases, preferably tumour pathologies, as e.g. melanoma, glioblastoma, colorectal carcinoma, breast carcinoma, kidney carcinoma, ovary carcinoma, cervical carcinoma, non-small cell lung carcinoma and/or metastasis, retinopathies as diabetic retinopathy, neovascularization in age-related exudative macular degeneration, macular oedema induced by occlusion of retina artery, choroid neovascularization caused by pathologic myopia, comprising administering to a patient in need thereof a therapeutically effective amount of the antibody or a synthetic or recombinant fragment thereof as defined above. It is a further object of the invention a pharmaceutical composition comprising at least the antibody or a synthetic or recombinant fragment thereof as defined above and pharmaceutical acceptable excipients, preferably said composition being for use by parenteral administration, in particular intravenously. The composition comprises an effective amount of the antibody and/or recombinant or synthetic antigen binding fragments thereof. The pharmaceutical compositions are conventional in this field and can be produced by the skilled in the art just based on the common general knowledge. The formulations as described herein typically comprise the antibody or synthetic or recombinant antigen binding fragments thereof as described above, in a concentration from about 0.1 mg/ml to about 100 mg/ml, preferably from 0.1 to 10 mg/ml, more preferably from 0.1 to 5 mg/ml. The antibody of the invention is administered to the patient in one or more treatments. Depending on the type and severity of the disease, a dosage of about 1 mg/kg to 20 mg/kg of the antibody or synthetic or recombinant antigen binding fragments thereof may be administered, for example in one or more administrations, or by continuous infusion. The antibodies of the present invention may be administered in combination with other therapeutic agents, in particular with antibodies able to neutralize other receptors involved in tumour growth or angiogenesis. Any method of administration may be used to administer the antibody of the present invention, in particular, for example, the administration may be oral, intravenous, intraperitoneal, subcutaneous, or intramuscular. The antibody according to the present invention may also be administered as a conjugate, which binds specifically to the receptor and releases toxic substances. In particular embodiments, the pharmaceutical composition of the present invention can be administered in the form of single dosage (for example, tablet, capsule, bolus, etc..). For pharmaceutical applications, the composition may be in the form of a solution, for example, of an injectable solution, emulsion, suspension, or the like. The vehicle can be any vehicle suitable from the pharmaceutical point of view. Preferably, the vehicle used is capable of increasing the entry effectiveness of the molecules into the target cell. In the pharmaceutical composition according to the invention, the antibody may be associated with other therapeutic agents, such as antagonists of other growth factor receptors involved in tumorigenesis or angiogenesis, such as VEGFR-2, EGFR, PDGFR, receptor kinase inhibitors, BRAF inhibitors, MEK inhibitors, immunomodulatory antibodies, anticancer agents, such as: bevacizumab, ramucirumab, aflibercept, sunitinib, pazopanib, sorafenib, cabozantinib, axitinib, regorafenib, nintedanib, lenvatinib, vemurafenib, dabrafenib, trametinib, chemotherapeutic agents such as methylating agents (temozolomide, dacarbazine), platinum compounds (cisplatin, carboplatin, oxaliplatin), taxanes (paclitaxel, nab-paclitaxel, docetaxel), fluoropyrimidines (5-fluorouracil, capecitabine), topoisomerase I inhibitors (irinotecan, topotecan), poly(ADP-ribose) polymerase inhibitors (PARP) (e.g., olaparib), etc. The pharmaceutical composition is chosen according to the demands of treatment. These pharmaceutical compositions according to the invention may be administered in the form of tablets, capsules, oral preparations, powders, granules, pills, liquid solutions for injection or infusion, suspensions, suppositories, preparations for inhalation. A reference for the formulations is the book by Remington ("Remington: The Science and Practice of Pharmacy", Lippincott Williams & Wilkins, 2000). The skilled in the art will choose the form of administration and the effective dosages, by selecting suitable diluents, adjuvants and/or excipients.

The term "pharmaceutical composition" refers to a preparation that is in such a form as to permit to the biological activity of an active ingredient contained therein to be effective, and which contains no additional components which are unacceptably toxic to a subject to which the formulation may be administered. It is a further aspect of the invention a method for producing the antibody or a synthetic or recombinant fragment thereof as defined above, comprising the steps of culturing the host cell and purifying the antibody or a synthetic or recombinant fragment thereof from the cell culture.

### DETAILED DESCRIPTION OF THE INVENTION

The present invention will be described by means of examples, referring to the following figures:

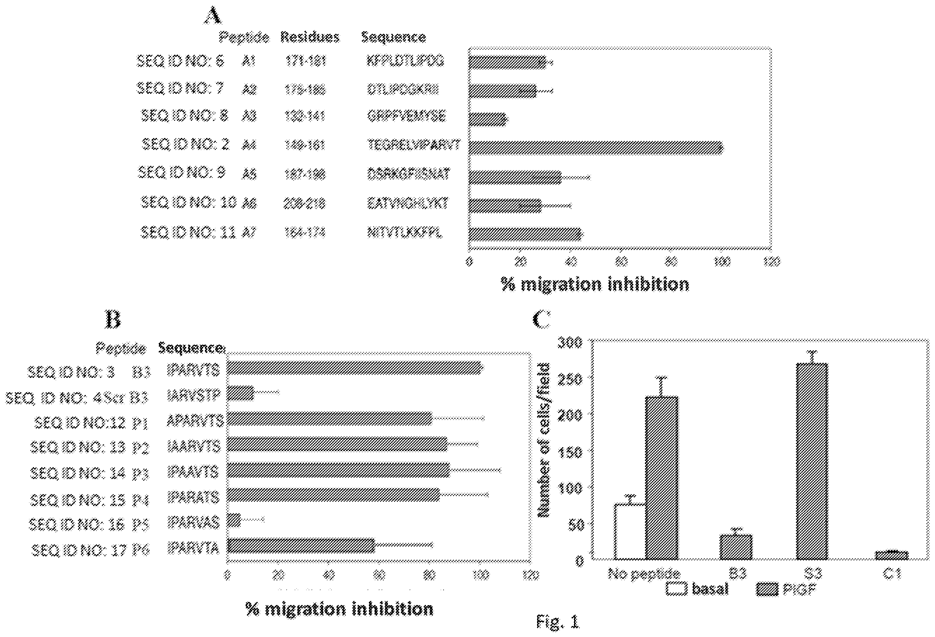

**Fig. 1****. Identification of a region of the domain II of VEGFR-1 involved in the activation of this receptor** [Lacal 2008].
   A) The peptide A4 (SEQ ID No. 2), consisting of a modified fragment of the Ig-like domain II of VEGFR-1, inhibits the migration of human endothelial cells. The activity of the indicated peptides (30 µM) was measured as inhibitory capacity of migration in response to the stimulation of endothelial cells with 5 µg/ml sVEGFR-1 in Boyden chambers provided with filters coated with gelatin. Data are expressed as percent inhibition of migration +/- standard error of the mean (SD).
   B) Identification of the amino acid residues of peptide B3 (SEQ ID No. 3) involved in its biological activity. The activity of the peptides was measured as inhibitory capacity of migration in response to the stimulation of endothelial cells with PIGF (50 ng/ml) in Boyden chambers provided with filters coated with gelatin. The peptides B3 and S3 [Scr B3= S3, SEQ ID No. 4] were included in the assay as positive and negative controls, respectively. Data are expressed as percent inhibition of migration +/- SD.
   C) Effect of the peptide C1 (RVTS) (SEQ ID No. 5) on the migration of endothelial cells. The inhibitory capacity on the migration in response to stimulation of endothelial cells with PIGF (50 ng/ml) by the peptide C1 (30 µM), was evaluated using Boyden chambers provided with filters coated with gelatin. The peptides B3 and S3 were included in the assay, at the same concentration of C1, as positive and negative controls, respectively. Data are expressed as mean number of cells per microscopic field (x200) +/- SD.
   Each value was calculated by examining three filters for a total of 10 microscopic fields.

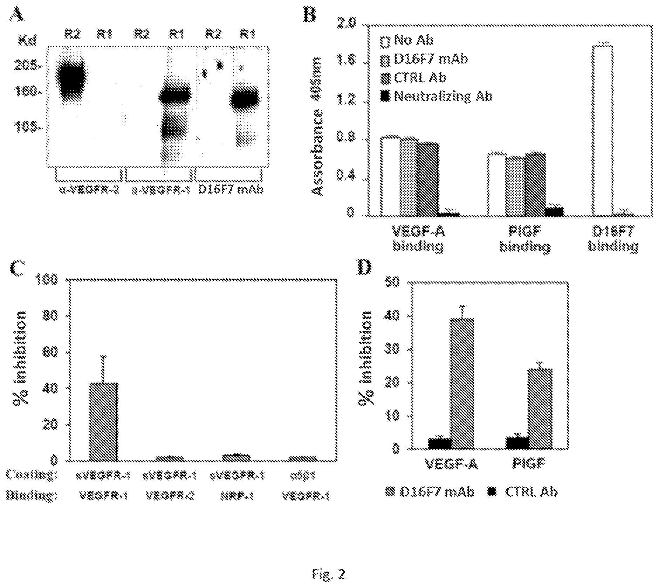

**Fig. 2****. Characterization of the D16F7 mAb.**
   A) Recognition of VEGFR-1 by mouse D 16F7 mAb. The ability of D16F7 mAb of specifically recognizing the extracellular region of VEGFR-1 was confirmed by Western blotting using the chimeric proteins VEGFR-1/Fc (R-1) and VEGFR-2/Fc (negative control, R-2). Commercial polyclonal antibodies specific for VEGFR-1 (AF321, R&D Systems) or VEGFR-2 (AF357, R&D Systems) were used as controls.
   B) The monoclonal antibody D16F7 does not interfere with the binding of PlGF or VEGF-A to VEGFR-1. Selected wells of 96-well plates coated with the chimeric protein sVEGFR-1/Fc were pre-incubated with D16F7 mAb or with an independent mouse IgG as a negative control (Ab CTRL). VEGF-A or PIGF were then added to the wells, either alone (VEGF-A or PlGF binding) or in combination with neutralizing anti-VEGF-A or anti-PlGF antibodies. After 2 hours of incubation, the plates were washed, and biotinylated anti-PlGF or anti-VEGF-A antibodies were added to the appropriate wells. The amount of VEGF-A or PlGF linked to the chimeric sVEGFR-1/Fc was determined by incubating the plates with streptavidin conjugated with alkaline phosphatase and measuring the alkaline phosphatase reaction as an absorbance at 405 nm. It was also assessed the binding of D16F7 mAb to the chimeric protein sVEGFR-1/Fc (D16F7 binding). Each value represents the mean +/- SD of three independent determinations.
   C) Effect of D16F7 mAb on the formation of homodimers of VEGFR-1 or heterodimers with other VEGF-A receptors, or with the integrin α5β1. Wells selected from 96-well plates, coated with sVEGFR-1 or integrin α5β1, were pre-incubated with D16F7 mAb or with a independent mouse IgG as a negative control (Ab CTRL), and then treated with chimeric proteins containing the extracellular regions of the VEGF-A receptors and a portion of the human Fc. After 2 hours of incubation, the plates were washed and the amount of chimera bound to sVEGFR-1 or to integrin α5β1 was determined by incubating the plates with a human anti-Fc antibody conjugated with alkaline phosphatase and measuring the alkaline phosphatase reaction as an absorbance at 405 nm. The effect of pre-incubation with D16F7 mAb was expressed as percent binding inhibition. D) Effect of D16F7 mAb on phosphorylation of VEGFR-1, analysed using an ELISA assay (R&D Systems) and extracts of human melanoma cells CR-Mel stimulated with VEGF-A or PlGF (200 ng/ml), after preincubation in the presence of 10 µg/ml of mAbD16F7 or an independent mouse IgG (Ab CTRL).

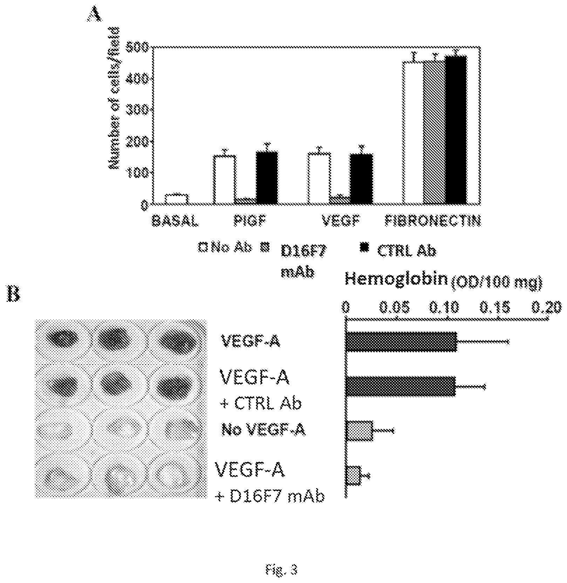

**Fig. 3****. In vitro and in vivo effect of D16F7 mAb on endothelial cells.**
   A) The monoclonal antibody D16F17 inhibits the migration of endothelial cells. The effect of antibody D16F17 (5 µg/ml) on the migration of HUV-ST endothelial cells was evaluated using Boyden chambers provided with filters coated with gelatin. As migratory stimuli, PlGF and VEGF-A (both at 50 ng/ml) were used, as specific stimuli for VEGFR-1, or fibronectin (5 µg/ml) as nonspecific stimulus. An irrelevant mouse antibody (Ab CTRL) was included in the assay as a negative control. Data are expressed as mean number of cells per microscopic field (x200) +/- SD. Each value was calculated by examining three filters for a total of 10 microscopic fields. B) The monoclonal antibody D16F7 inhibits the neovascularization, as assessed in matrigel assays in vivo. Five hundred µl of matrigel containing VEGF-A (100 ng/ml) as a angiogenic stimulus (positive control) or in the absence of VEGF-A (negative control) were injected subcutaneously in the flank of C57BL/6 mice, and five days after the injection the matrigel was extracted from mouse and photographed. In some samples containing the angiogenic stimulus, the D16F7 mAb or a control mouse IgG (5 µg/ml) were included. The hemoglobin content in the matrigel pellet was determined by spectrophotometer using Drabkin's reagent. The values express the mean optical density (OD)/100 mg matrigel +/- SD.

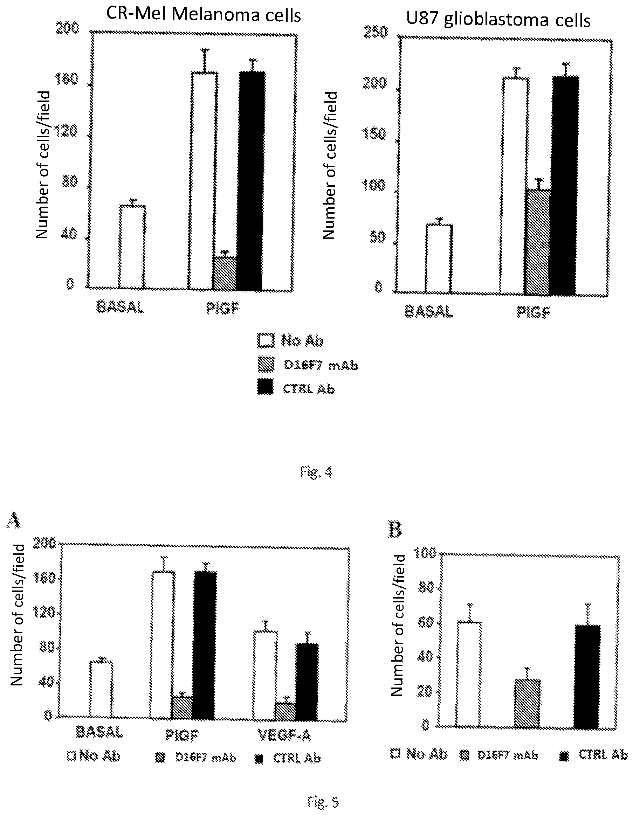

**Fig. 4****. Effect of pretreatment with D16F17 mAb on the migration of human cancer cells.**
   The effect of antibody D16F17 (5 µg/ml) on the migration of human melanoma cells (CR-Mel) or glioblastoma cells (U87) was evaluated using Boyden chambers provided with filters coated with gelatin. PlGF (50 ng/ml) has been used as a migratory stimulus. An irrelevant mouse antibody (Ab CTRL) was included in the assay as a negative control. Data are expressed as mean number of cells per microscopic field (x200) +/- SD. Each value was calculated by examining three filters for a total of 10 microscopic fields.
**Fig. 5****. Effect of pretreatment with D16F17 mAb on the invasiveness of melanoma cells.**
   A) The monoclonal antibody D16F17 inhibits the migration of melanoma cells in response to PlGF and VEGF-A. The effect of the antibody D16F17 (5 µg/ml) on the migration of CR-Mel cells was evaluated using Boyden chambers provided with filters coated with gelatin. PIGF or VEGF-A (50 ng/ml) were used as migratory stimuli. An irrelevant mouse antibody (Ab CTRL) was included in the assay as a negative control. Data are expressed as mean number of cells per microscopic field (x200) +/- SD. Each value was calculated by examining three filters for a total of 10 microscopic fields. B) The monoclonal antibody D16F7 inhibits the invasion of the extracellular matrix by the CR-Mel melanoma cells. The invasion tests were carried out in Boyden chambers by using filters coated with 20 µg of matrigel. The invasiveness of CR-Mel cells through the extracellular matrix was assessed in the absence or in the presence of 5 µg/ml of the monoclonal antibody D16F7 (D16F7 mAb) or a control mouse IgG (Ab CTRL). The baseline invasion (in the absence of stimuli) was expressed as the mean number of cells per microscopic field (x200) standard deviation of the mean. Each value was calculated by examining three filters for a total of ten microscopic fields.

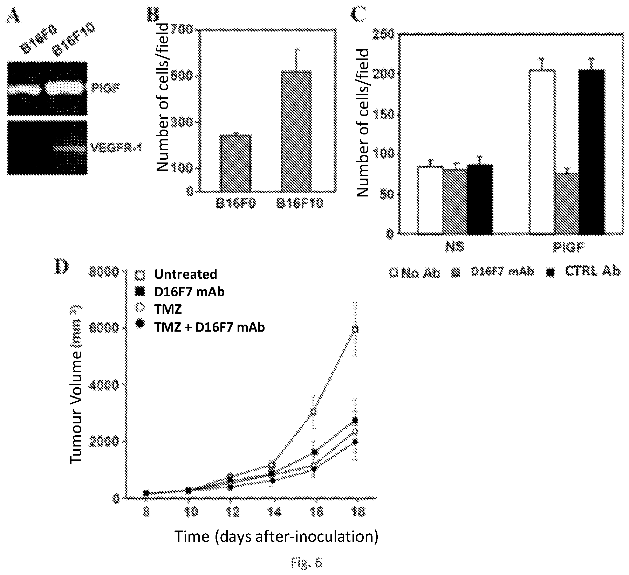

**Fig. 6****. Effect of the administration of D16F7 mAb on tumour growth in a mouse model of melanoma in vivo.**
   A) Expression of PlGF and VEGFR-1 in two clones of mouse melanoma line B 16 (clones F0 and F10) evaluated by RT-PCR. B) In vitro analysis of the invasive ability of the two cell clones of the line B16, evaluated using Boyden chambers provided with filters coated with 20 µg of matrigel. C) Effect of the treatment with 5 µg/ml of D16F7 mAb on the ability of B16F10 cells to invade extracellular matrix in response to PlGF (50 ng/ml), evaluated using Boyden chambers provided with filters coated with 20 µg of matrigel. NS: no stimulus. D) Mouse melanoma cells B16F10 were implanted intramuscularly (i.m.) in male BDF1 mice (250,000 cells per animal). Once the tumour become detectable (i.e., 8 days after inoculation), the mice were treated with D16F7 mAb (10 mg/kg), temozolomide (TMZ, 100 mg/kg), or both, according to the following protocol: TMZ, 5 daily doses between day 8 and day 12; D16F7 mAb, every 48 hours from day 8 after inoculation up to the animal sacrifice. In the panels A-C, the data represent the values of the arithmetic mean +/- SD of three independent determinations.

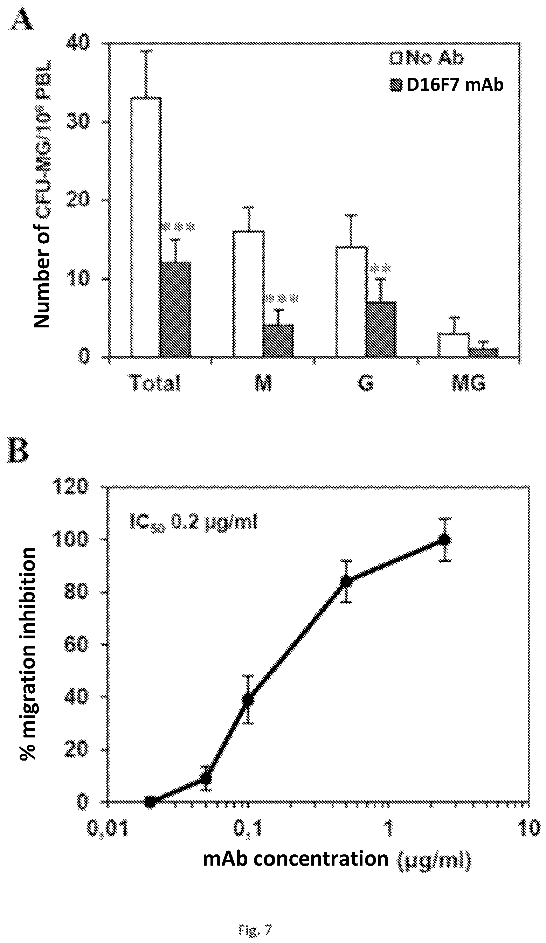

**Fig. 7****. Effect of the treatment with D16F17 mAb on cells of a myelomonocytic line**
   A) The treatment with the monoclonal antibody D16F7 reduces the number of circulating hematopoietic progenitors. BDF1 male mice inoculated i.m. with B16F10 mouse melanoma cells (250,000 cells per animal) were treated with D16F7 mAb (10 mg/kg) starting from day 8 after inoculation (when the cancer was detected), every 48 hours. After 8 days of treatment, from each of the control animals and from each of those treated with mAb (5 per group) 400 µl of blood were taken. Blood samples taken from the animals belonging to the same group were combined together. The quantification of the number of progenitors of granulocytes (G) and/or macrophages (M) circulating in the peripheral blood of the analysed mice was performed using a semi-solid medium that contains methylcellulose and recombinant cytokines that support the growth of colonies (CFU) from these precursors. The values shown are the average of the number of CFU-G, CFU-M and CFU-MG per million of peripheral blood leukocytes (Peripheral Blood Leukocytes, PBL) measured in 8 different plates for each experimental group (untreated or treated with the monoclonal antibody D16F7) +/- SD. ***, p< 0.001, **, p< 0.01. B) The monoclonal antibody D16F7 inhibits the migration of monocytic cells in response to PIGF. A dose-effect curve was established to assess the inhibition by the antibody D16F17 of the migration of differentiated HL60 cells to monocytes/macrophages by treatment with the differentiating agent PMA (culture in the presence of 10 ng/ml PMA for 24 hours). The migration was evaluated using Boyden chambers provided with filters coated with gelatin and 50 ng/ml of PlGF as a stimulus. The IC50 value was calculated using the software CalcuSyn.

### Materials and Methods

### Reagents

Endothelial Basal Medium (EBM-2) and Endothelial Growth Factor Medium (EGM-2) were purchased from Clonetics (BioWhittaker Inc, Walkersville, MD) and Fatty acid-free bovine serum albumin (BSA) from Roche (Mannheim, Germany). The culture media RPMI 1640 and DMEM, as well as the foetal bovine serum (FBS) were purchased from Sigma-Aldrich (St. Louis, MO). Matrigel (commercial extracellular matrix) was purchased from BD Biosciences (Bedford, MA). VEGF-A, PlGF, the antibodies specific for these cytokines, control mouse IgG and the chimeric proteins rhVEGFR-1/Fc, rhVEGFR-2/Fc, rrNeuropilin-1/Fc (NRP-1/Fc), were purchased from R&D Systems (Abingdon, UK). sVEGFR-1 was purchased from RELIATech (Braunschweig, Germany), fibronectin and gelatin from Sigma-Aldrich. The purified human integrin α5β1 and the anti-α5β1 integrin monoclonal antibody JBS5 were purchased from Chemicon (Temencula, CA), geneticin from Invitrogen (Groningen, Netherlands) and puromycin and other chemical reagents from Sigma-Aldrich.

### Cells

The cell line HUV-ST is an immortalized line obtained from endothelial cells from the human umbilical vein (WO2006067742A2, WO2006067742A3), and was generated and maintained in culture in EGM-2 medium supplemented with 0.4 mg/ml geneticin and 5 µg/ml puromycin as described previously (Tentori, 2005). The melanoma line CR-Mel has been produced in the Molecular Oncology Laboratory of the IDI-IRCCS, as previously described (Ruffini, 2011) and obtaining the informed consent from the patient, while the lines B16F0 (ATCC CRL-6322), B16F10 (ATCC CRL-6475), HL60 (ATCC CCL-240) and U87 (ATCC HTB-14) were obtained from ATCC. The melanoma cells and the HL60 cells were maintained in culture in medium RPMI1640 supplemented with 10% FBS, while the U87 cells in DMEM/10% FBS.

### Production of the monoclonal antibody D16F7

BALB/C mice (purchased from Charles River, Italy) were immunized with the peptide A4 in the form of MAP (Multiple Antigenic Peptide) using standard protocols. The fusion between splenocytes of immunized mice and the murine myeloma NSO-1 cells was carried out in 50% polyethylene glycol, and the selection of the hybridomas was carried out in medium supplemented with HAT. Using an ELISA assay, carried out using plates coated with a soluble form of VEGFR-1, a total of 12 hybridomas were selected which produce anti-VEGFR-1 monoclonal antibodies. Among these, the hybridoma D16F7 was selected for its ability to produce a monoclonal antibody capable of recognizing the extracellular region of VEGFR-1, but not of VEGFR-2, by Western blotting assays. This hybridoma was then cloned, and the clone obtained, that preserves the parental hybridoma characteristics, was selected for further characterization.

### Evaluation of the interaction of VEGFR-1 with itself (formation of homodimers) or with other polypeptides (formation of heterodimers)

The interaction of the extracellular region of VEGFR-1 with other cell surface proteins or with its ligands has been evaluated in 96-well plates Maxisorp Nunc immunoplates (Nunc, Roskilde, Denmark) coated with sVEGFR-1, using a 10 µg/ml solution of the polypeptide.

In the case of other cell surface proteins, chimeric polypeptides that contain the extracellular region of VEGFRs and the Fc region of human immunoglobulins were interacted with sVEGFR-1 on the plate in the presence or absence of the molecules to be analysed for their interfering capacity with such interactions. The binding of the chimeric protein was measured using an anti-human immunoglobulin antibody conjugated with alkaline phosphatase and subsequent alkaline phosphatase reaction detected as an absorbance at 405 nm. The interaction of VEGFR-1 with integrin α5β1 was assessed in a similar way, using wells coated with the purified integrin and the chimeric protein VEGFR-1/Fc.

As regards the assessment of the binding of VEGF-A and PlGF to VEGFR-1, selected wells coated with the chimeric protein VEGFR-1/Fc were incubated with 50 ng/ml VEGF-A or PIGF, either alone or in combination with neutralizing antibodies anti-VEGF-A or anti-PlGF. After 2 hours of incubation at room temperature, the plates were washed, and biotinylated anti-PlGF or anti-VEGF-A antibodies were added to the appropriate wells. The amount of VEGF-A or PIGF bound to the chimeric sVEGFR-1/Fc was then determined by incubating the plates with streptavidin conjugated with alkaline phosphatase (1:10000, Roche) and measuring the alkaline phosphatase reaction as the absorbance at 405 nm. The assessment of the effect of the molecules to be analyzed for their ability to interfere with the binding of VEGF-A or PlGF to VEGFR-1 was carried out by pre-incubating the plates coated with VEGFR-1 with the molecules of the present study.

### Evaluation of VEGFR-1 phosphorylation

Phosphorylation of membrane VEGFR-1 was analyzed using an ELISA assay and extracts of human melanoma cells CR-Mel stimulated with VEGF-A or PlGF (200 ng/ml). Cells (10⁶ cells/well) were seeded in 6-well plates. The next day, they were incubated for 5 hours in serum-free medium, with 0.1% BSA, at 37°C in a CO₂ incubator, pretreated with 10 µg/ml of monoclonal antibody D16F7 or control mouse IgG for 30 min at room temperature, and stimulated for 10 minutes at 37°C in a CO₂ incubator with 200 ng/ml of VEGF-A or PlGF. The determination of the amount of total (samples from 20 µg of extract) and phosphorylated (samples from 30 µg of extract) VEGFR-1 was then performed using a Human Phospho-VEGFR-1 DuoSet IC ELISA Kit (R&D Systems), as indicated in the kit manual.

### Migration assay in vitro

In vitro cell migration was evaluated using Boyden chambers provided with polycarbonate filters with a pore size of 8 µM in diameter (Nuclepore, Whatman Incorporated, Clifton, NJ) (Orecchia, 2003). In chemotaxis assays, cells were harvested, resuspended in migration medium (0.1% BSA in EBM-2 medium) and loaded (1.5×10⁵ cells) in the upper compartment of Boyden chambers provided with filters coated with a 5 µg/ml solution of gelatin. In the samples in which the VEGFR-1 inhibitory effect of molecules on migration has to be evaluated, the cells were pretreated with inhibitors for 30 minutes at room temperature before the test and then loaded directly in the Boyden chamber along with the inhibitor. Migration medium was added in the lower compartment of the chambers, either alone or containing the chemotactic stimulus. For each experimental condition three chambers were employed. After incubation of the samples at 37°C in a CO₂ incubator for 18 hours, the filters were removed from the chambers, the cells were fixed with 100% ethanol and stained in 0.5% crystal violet. The cells attached to the upper surface of the filter were removed and those that had migrated, adhered to the lower surface, were counted under a microscope. For each experimental condition twelve microscopic fields were counted (magnification x200), of filters in triplicate. A similar procedure was used for the invasion assays of the extracellular matrix, using filters coated with matrigel and 4-hour incubation times.

### Angiogenesis in vivo

Five hundred µl of matrigel containing VEGF-A (100 ng/ml) as an angiogenic stimulus or without VEGF-A (negative control) were injected subcutaneously in the flank of C57BL/6 mice (Charles River Laboratories, Calco, Italy). In some samples containing the angiogenic stimulus, the D16F7 mAb or a control antibody (5 µg/ml) were included. Five days after injection, the animals were sacrificed, the matrigel was extracted and photographed for a macroscopic analysis of angiogenesis. The hemoglobin content in the matrigel pellet was measured in a spectrophotometer using the Drabkin method (Biroccio, 2000). All procedures that required the use of animals were carried out in accordance with international guidelines (Council Directives of the European Economic Community 86/109, OLJ318, Dec. 1.1987).

### RT-PCR

The reverse transcriptase polymerase chain reaction (RT-PCR) was performed from total RNA obtained from different analyzed cell lines, using a RNeasy Midi kit from Qiagen (Hilden, Germany), according to the kit manual.

Three micrograms of total RNA per sample were used for reverse transcription reaction by the enzyme from avian myeloblastosis virus (AMV, Boehringer Mannheim, Mannheim, Germany) for 60 minutes at 42°C in a 25 µl volume. Five µl of the cDNA preparation were used for each amplification reaction with 1 U of Dynazyme II DNA polymerase (Finnzymes OY, Espoo, Finland). The primers and hybridization conditions were:
forward primer, 5'-GCACCAAGAGCGATGTGTGG-3' (SEQ ID No. 18) and
reverse primer, 5'-ACACCACGGAGTTGTAGTCT-3' (SEQ ID No. 19)
for mouse VEGFR-1 (hybridization conditions of 30 s at 58°C);
forward primer, 5'-ATTTCCCCTGCTCGTACCACT-3' (SEQ ID No. 20) and
reverse primer, 5'-TCACGGGTGGGGTTCCTCA-3' (SEQ ID No. 21)
for mouse PlGF (hybridization conditions of 30 s at 58°C).

### Evaluation of the effect in vivo of the monoclonal antibody D16F7 in a syngeneic model of mouse melanoma

B16F10 mouse melanoma cells were implanted s.c. in BDF1 male mice (Charles River, Italy) (250,000 cells per animal). Once the tumour became detectable (i.e., 8 days after inoculation), mice were treated with D16F7 mAb (10 mg/kg), temozolomide (TMZ, 100 mg/kg), or both, according to the following protocol: TMZ, 5 daily doses between day 8 and day 12; D16F7 mAb, every 48 hours from day 8 after inoculation up to the animal sacrifice. The melanoma growth was monitored by measuring tumour mass with a calliper every 2 days. Tumour volume was calculated using the following formula: volume = [(width)² × length]/2. All handling and housing procedures of the animals were carried out in accordance with international guidelines and directives (Directive 2010/63/EU of the European Economic Community Council and the European Parliament).

The fact that VEGFR-1 is expressed in cells of the myelomonocytic line and that its expression promotes chemotaxis of these cells suggested a possible effect of the monoclonal antibody D16F7 on the mobilization of monocyte/macrophage precursors in treated animals. This aspect has been analyzed using a method for detecting the haematopoietic progenitors in the peripheral blood of the animals on a semi-solid medium containing methylcellulose (MethoCult 3534). Such medium promotes optimal growth of granulocyte/macrophage progenitors (Colony-Forming Unit-Granulocyte, Macrophage [CFU-GM]). For this purpose, 400 µl of peripheral blood were taken from each mouse using test tubes containing 20 IU/ml of heparin. For each experimental group (control and treated with the antibody D16F7), a collection of peripheral blood has been carried out from 5 animals per group, for a total of 2 ml. The erythrocytes were then lysed using an ammonium chloride solution and leukocytes were resuspended in a small volume of medium. Cell suspension was then mixed with MethoCult and 1 ml of the cells/support mixture loaded on 3.5 cm diameter Petri dishes, following the indications of the product manual. After 12 days, the CFUs of precursors of granulocytes, macrophages, or both types, have been identified and counted under a microscope as indicated in the MethoCult manual.

### Sequencing of the monoclonal antibody D16F7

The sequencing of the antibody was carried out starting from hybridoma cells producing it. Total RNA was extracted from the cells using the TRIzol^{®} Plus RNA Purification System and cDNA was synthesized using antisense primers specific for the IgG1 isotype using a SuperScriptTM III First-Strand Synthesis System. The variable regions of the heavy and light chains of the antibody were amplified by RT-PCR and cloned separately into standard cloning vectors. After the analysis of the colonies by PCR to identify the positive ones, ten colonies for each antibody fragment were sequenced to derive a consensus sequence of the variable regions of antibody D16F7, which are described below.

The variable region of the heavy chain has a coding sequence of 399 bp corresponding to an amino acid sequence of 133 aa, according to the following scheme:

### Leader Sequence-FR1-CDR1-FR2-CDR2-FR3-CDR3-FR4

SEQ ID No. 23
MGWSSIILFLVATASGVHSQVQLQQPGSELVRPGASVKLSCKASGYIFTNYWMHWVKQ
RPGQGLEWIGEIYPRNGNTNYDEKFKGKATLTIDTSSSTAYMQLSSLTSEDSAVYYC
ATYLGDYWGQGTTLTVSS

SEQ ID No. 22
ATGGGATGGAGCTCTATCATCCTCTTCTTGGTAGCAACAGCCTCAGGTGTCCACTCCCAG
GTCCAACTGCAGCAACCTGGGTCTGAACTGGTGAGGCCTGGAGCTTCAGTGAA
GCTGTCCTGCAAGGCTTCTGGCTACATATTCACCAACTACTGGATGCACTGGGTG
AAGCAGAGGCCTGGACAAGGCCTTGAGTGGATTGGAGAAATTTATCCTCGTAAT
GGTAATACTAACTACGATGAGAAGTTCAAGGGCAAGGCCACACTGACTATAGACA
CATCCTCCAGCACAGCCTACATGCAGCTCAGCAGCCTGACATCTGAGGACTCT
GCGGTCTATTACTGTGCCACCTACCTAGGGGACTACTGGGGCCAAGGCACCACT
CTCACAGTCTCCTCA

The variable region of the light chain has a coding sequence of 393 bp corresponding to an amino acid sequence of 131 aa, according to the following scheme:

### Leader Sequence-FR1-CDR1-FR2-CDR2-FR3-CDR3-FR4

SEQ ID No. 25

MKLPVRLLVLMFWIPASSSDVLMTQTPLSLPVSLGDQASISCRSSQSIVHSNGNTYLEWF
LQKPGQSPKLLIYKVSNRFSGIPDRFSGSGSGTDFTLKISRVEAEDLGVYFCFQGSHV
PYTFGGGTKLEIK

SEQ ID No. 24
ATGAAGTTGCCTGTTAGGCTGTTGGTGCTGATGTTCTGGATTCCTGCTTCCAGCAGTGAT
GTTTTGATGACCCAAACTCCACTCTCCCTGCCTGTCAGCCTTGGAGATCAAGCC
TCCATCTCTTGCAGATCTAGTCAGAGTATTGTTCATAGTAATGGAAACACCTATTTA
GAATGGTTCCTGCAGAAACCAGGCCAGTCTCCAAAGCTCCTGATCTACAAAGTT
TCCAACCGATTTTCTGGGATCCCAGACAGGTTCAGTGGCAGTGGATCAGGGACA
GATTTCACACTCAAGATCAGTAGAGTGGAGGCTGAGGATCTGGGAGTTTATTT
CTGCTTTCAAGGTTCACATGTTCCGTACACGTTCGGAGGGGGGACCAAGCTGGAA
ATAAAA

### Cell proliferation

Cell proliferation was assessed in 96-well plates using a reduction assay of the tetrazolium derivative MTS [3-(4,5-dimethylthiazol-2-yl)-5-(3-carboxy-methoxyphenyl)2-(4-sulfophenyl)-2H-tetrazolium] (Promega, Madison, WI) (Promega, Madison, WI), quantified by measuring the absorbance at 490 nm, which is directly proportional to the number of living cells in the culture.

### Cell adhesion

The cell adhesion to different components of the extracellular matrix has been analyzed in 96-well plates coated with the different polypeptides (collagen, fibronectin, gelatin and matrigel). The adhesion efficacy on the extracellular matrix components after 30 min at 37°C was evaluated by staining adherent cells with crystal violet and measurement in a spectrophotometer of the extracted dye from the same cells.

### RESULTS

### Peptides A4 and B3

A peptide of 13 amino acids was identified (A4, SEQ ID No. 2) comprised in the II immunoglobulin-like domain of VEGFR-1 and having a mutated amino acid **(****Figure 1A****),** capable of blocking the migration of human endothelial cells as induced by VEGF-B (also capable of binding VEGFR-1) and by PIGF, without interfering with the receptor binding of the growth factors. A peptide of 7 amino acids (B3, SEQ ID. No.3), derived from the peptide A4, retains the ability of the latter to block the migration induced by VEGF-B and PlGF **(****Figure 1B****, 1C),** proving to contain the amino acid sequence responsible for the inhibitory activity of the peptide A4 on the chemotaxis of endothelial cells (Lacal, 2008). In addition, the peptide B3 inhibits angiogenesis both *in vitro* (vascular structure formation assay in matrigel) and *in vivo* (vascularization assay of matrigel implanted subcutaneously in healthy mice) (Lacal, 2008). The peptide B3 acts by binding specifically to VEGFR-1 receptor and interfering with its dimerization (Lacal, 2008). The peptide can block, at an early stage, the signal transduced upon binding of the growth factor to the membrane VEGFR-1. Because the peptide B3 does not block the binding of growth factors to VEGFR-1 (Lacal, 2008), it does not interfere with the known ability of endogenous soluble VEGFR-1 to sequester VEGF and PlGF and so to prevent the activation of the membrane VEGFR-1 and VEGFR-2 receptors.

### Monoclonal antibody D16F7

Using the peptide A4 as an antigen, a mouse monoclonal antibody (D16F7 mAb, an IgG1) has been produced that has anti-angiogenic and anti-tumour properties similar to the peptide B3. In particular:
a) it inhibits the dimerization and autophosphorylation of VEGFR-1 **(****Fig. 2C** and **2D****),** while not blocking the binding of VEGF-A or PlGF to VEGFR-1 **(****Fig. 2B****);**
b) it inhibits the migration of human endothelial cells (i.e., HUV-ST) induced by PlGF and VEGF-A **(****Fig. 3A****)** and has a strong anti-angiogenic activity in *in vivo* assays **(****Fig. 3B****);**
c) it inhibits the migration of human tumour cells that express VEGFR-1, and in particular melanoma and glioblastoma cells **(****Fig. 4 and** 5);
d) it inhibits the invasion of the extracellular matrix by melanoma cells in basal conditions **(****Fig. 5****).**

### In vivo data

The monoclonal antibody D16F7 has proved to be effective also in a syngeneic mouse model of melanoma (melanoma line B16F10 inoculated s.c. in B6D2F1 male mice, **Fig. 6****).** This allowed to perform *in vivo* preclinical studies aimed at evaluating a therapeutic potential of the antibody. Intraperitoneal administration of the monoclonal antibody D16F7 at doses up to 40 mg/kg in C57BL/6 mice every 48 hours, for a total of 5 doses, are well tolerated, since it was not observed any signs of systemic toxicity after treatment. Also, antibody serum levels of the animals are maintained largely higher than the concentrations at which anti-angiogenic and antitumor activity is detected *in vitro,* for at least one week after the last dose: 200-300 µg/ml for doses of 10 mg/kg and 1-2 mg/ml for doses of 40 mg/kg.

In this syngeneic mouse model, the treatment with 10 mg/kg of antibody D16F7 every 48 hours results in a strong anti-tumour effect, comparable to that of temozolomide, a drug used in the treatment of metastatic melanoma **(****Fig. 6D****),** and also significantly reduces the number of circulating hematopoietic myeloid progenitors **(****Fig. 7A****).** The antibody effect on the migration of cells of myeloid line was confirmed using the human promyelocytic line HL60 after differentiation into monocytic cells by treatment with phorbol 12-myristate 13-acetate (PMA). The differentiation promotes the expression of VEGFR-1 into these cells, and the antibody D16F7 inhibits also their migration induced by PlGF **(****Fig. 7B****).**

Instead, *in vitro* assays have not revealed any effects of the antibody on the ability of endothelial and melanoma cells to adhere to extracellular matrix components or on the proliferation of melanoma cells, both in the baseline conditions and under stimulation with PIGF.

### REFERENCES

Lohela M, Bry M, Tammela T, Alitalo K. VEGFs and receptors involved in angiogenesis versus lymphangiogenesis. Curr Opin Cell Biol 21: 154-165, 2009
Kendall RL, Wang G, and Thomas KA. Identification of a natural soluble form of the vascular endothelial growth factor receptor, FLT-1, and its heterodimerization with KDR. Biochem Biophys Res Commun 226: 324-328, 1996
Ruffini F, Failla CM, Orecchia A, Bani MR, Dorio AS, Fortes C, Zambruno G, Graziani G, Giavazzi R, D'Atri S, Lacal PM. Expression of the soluble vascular endothelial growth factor receptor-1 in cutaneous melanoma: role in tumour progression. Br J Dermatol 164: 1061-1070, 2011
Fischer C, Mazzone M, Jonckx B, Carmeliet P. FLT1 and its ligands VEGFB and PIGF: drug targets for anti-angiogenic therapy? Nature Reviews 8: 942-956, 2008
Norden AD, Drappatz J, Wen PY. Antiangiogenic therapies for high-grade glioma. Nat Rev Neurol 5: 610-620, 2009
Higa GM, Abraham J.Biological mechanisms of bevacizumab-associated adverse events. Exp Rev Anticancer Ther 9: 999-1007, 2009
Tentori L Vergati M, Muzi A, Levati L, Ruffini F., Forini O, Vernole P, Lacal PM, Graziani G. Generation of an immortalized human endothelial cell line as a model of neovascular proliferating endothelial cells to assess chemosensitivity to anticancer drugs. Int J Oncol 27: 525-535, 2005
Orecchia A., Lacal PM, Schietroma C, Morea V, Zambruno G, and Failla CM. Vascular endothelial growth factor receptor-1 is deposited in the extracellular matrix by endothelial cells and is a ligand for the α5β1 integrin. J Cell Sci 116: 3479-3489, 2003
Biroccio A, Candiloro A, Mottolese M, Sapora O, Albini A, Zupi G, Del Bufalo D. Bcl-2 overexpression and hypoxia synergistically act to modulate vascular endothelial growth factor expression and in vivo angiogenesis in a breast carcinoma line. FASEB J 14: 652-660, 2000
Lacal PM, Morea V, Ruffini F, Orecchia A, Dorio AS, Failla CM, Soro S, Tentori L, Zambruno G, Graziani G, Tramontano A, D'Atri S. Inhibition of endothelial cell migration and angiogenesis by a vascular endothelial growth factor receptor-1 derived peptide. Eur J Cancer 44:1914-1921, 2008
Dewerchin M and Carmeliet P. Placental growth factor in cancer. Expert Opin Ther Targets. 18:1339-1354, 2014
Arjaans M, Schröder CP, Oosting SF, Dafni U, Kleibeuker JE, de Vries EG. VEGF pathway targeting agents, vessel normalization and tumor drug uptake: from bench to bedside. Oncotarget doi: 10.18632/oncotarget.6918
Karimkhani C, Gonzalez R, Dellavalle RP. A review of novel therapies for melanoma. Am J Clin Dermatol 15:323-337, 2014.
Villani ME, Morea V, Consalvi V, Chiaraluce R, Desiderio A, Benvenuto E, Donini M. Humanization of a highly stable single-chain antibody by structure-based antigen-binding site grafting. Mol Immunol 45:2474-2485, 2008.
Spagnolo F, Picasso V, Lambertini M, Ottaviano V, Dozin B, Queirolo P. Survival of patients with metastatic melanoma and brain metastasis in the era of MAP-kinase inhibitors and immunologic checkpoint blockade antibodies: A systematic review. Cancer Treat Rev 45: 38-45, 2016.

## Claims

1. An antibody or a synthetic or recombinant fragment thereof able to recognize and bind to an epitope comprised in the sequence from aa. 149 to aa. 161 of SEQ ID No. 1 of VEGFR-1, and not to prevent the binding of VEGF-A or PlGF to VEGFR-1, comprising:
- a complementarity determining region 3 of the heavy chain (CDRH3) having the sequence YLGDY (aa. 118-122 of SEQ. ID No. 23), and
- a complementarity determining region 2 of the heavy chain (CDRH2) having the sequence EIYPRNGNTNYDEKFKG (aa. 69-85 of SEQ. ID No. 23), and
- a complementarity determining region 1 of the heavy chain (CDRH1) having the sequence GYIFTNYWMH (aa. 45-54 of SEQ. ID No. 23), and
- a complementarity determining region 3 of the light chain (CDRL3) having the sequence FQGSHVPYT (aa. 113-121 of SEQ. ID No. 25), and
- a complementarity determining region 2 of the light chain (CDRL2) having the sequence KVSNRFS (aa. 74-80 of SEQ. ID No. 25), and
- a complementarity determining region 1 of the light chain (CDRL1) having the sequence RSSQSIVHSNGNTYLE (aa. 43-58 of SEQ. ID No. 25).

2. The antibody or a synthetic or recombinant fragment thereof according to claim 1 able to recognize and bind to an epitope comprised in the sequence from aa. 156 to aa. 161 of SEQ ID No. 1 of VEGFR-1, and not to prevent the binding of VEGF-A or PlGF to VEGFR-1.

3. The antibody or a synthetic or recombinant fragment thereof according to claim 1 or 2 **characterized in that** it is obtained making use of the peptide of sequence TEGRELVIPARVT (SEQ ID No. 2).

4. The antibody or a synthetic or recombinant fragment thereof according to any of previous claims comprising:
- the variable region of the heavy chain comprising essentially the amino acid sequence:
QVQLQQPGSELVRPGASVKLSCKASGYIFTNYWMHWVKQRPGQGLEWIGEIYPRNGNTN YDEKFKGKATLTIDTSSSTAYMQLSSLTSEDSAVYYCATYLGDYWGQGTTLTVSS (aa. 20-133 of SEQ ID No. 23) and/or
- the variable region of the light chain comprising essentially the amino acid sequence:

DVLMTQTPLSLPVSLGDQASISCRSSQSIVHSNGNTYLEWFLQKPGQSPKLLIYKVSNRFSGI
PDRFSGSGSGTDFTLKISRVEAEDLGVYFCFQGSHVPYTFGGGTKLEIK (aa. 20-131 of
SEQ ID No. 25).

5. The antibody or a synthetic or recombinant fragment thereof according to any of previous claims, wherein said antibody is a monoclonal, chimeric, humanized or deimmunized antibody.

6. The antibody or a synthetic or recombinant fragment thereof according to any of previous claims, for use as medicament, preferably for use in the treatment and/or prevention of angiogenesis related pathologies, more preferably tumor pathologies, as e.g. melanoma, glioblastoma, colorectal carcinoma, breast carcinoma, kidney carcinoma, ovary carcinoma, cervical carcinoma, non-small cell lung carcinoma and/or metastasis, retinopathies as diabetic retinopathy, neovascularization in age-related exudative macular degeneration, macular oedema induced by occlusion of retina artery, choroid neovascularization caused by pathologic myopia.

7. A nucleic acid molecule encoding the antibody or a synthetic or recombinant fragment thereof as defined in claims 1-6, preferably comprising a nucleotide sequence essentially consisting of: 
CAGGTCCAACTGCAGCAACCTGGGTCTGAACTGGTGAGGCCTGGAGCTTCAGTGAAGC
TGTCCTGCAAGGCTTCTGGCTACATATTCACCAACTACTGGATGCACTGGGTGAAGCAG
AGGCCTGGACAAGGCCTTGAGTGGATTGGAGAAATTTATCCTCGTAATGGTAATACTA
ACTACGATGAGAAGTTCAAGGGCAAGGCCACACTGA
CTATAGACACATCCTCCAGCACAGCCTACATGCAGCTCAGCAGCCTGACATCTGAGGA
CTCTGCGGTCTATTACTGTGCCACCTACCTAGGGGACTACTGGGGCCAAGGCACCACTC
TCACAGTCTCCTCA (nt. 58-399 of SEQ ID No. 22)
 and/or

GATGTTTTGATGACCCAAACTCCACTCTCCCTGCCTGTCAGCCTTGGAGATCAAGCCTC
CATCTCTTGCAGATCTAGTCAGAGTATTGTTCATAGTAATGGAAACACCTATTTAGAAT
GGTTCCTGCAGAAACCAGGCCAGTCTCCAAAGCTCCTGATCTACAAAGTTTCCAACCG
ATTTTCTGGGATCCCAGACAGGTTCAGTGGCAGTGGATCAGGGACAGATTTCACACTC
AAGATCAGTAGAGTGGAGGCTGAGGATCTGGGAGTTTATTTCTGCTTTCAAGGTTCAC
ATGTTCCGTACACGTTCGGAGGGGGGACCAAGCTGGAAATAAAA (nt. 58-393 of SEQ ID
No. 24).

8. An expression vector encoding the antibody or a synthetic or recombinant fragment thereof as defined in claims 1-6, preferably comprising the nucleic acid as defined in claim 7.

9. A host cell comprising the nucleic acid according to claim 7, or the vector according to claim 8.

10. A pharmaceutical composition comprising at least the antibody or a synthetic or recombinant fragment thereof as defined in claims 1-6 and pharmaceutical acceptable excipients, preferably said composition being for use by parenteral administration, preferably intravenously.

11. A method to produce the antibody or a synthetic or recombinant fragment thereof as defined in claims 1-6 comprising the steps of culturing the host cell of claim 9 and purifying the antibody or a synthetic or recombinant fragment thereof from the cell culture.

## Patentansprüche

1. Antikörper oder synthetisches oder rekombinantes Fragment davon, der/das in der Lage ist, ein Epitop, das in der Sequenz von aa 149 bis aa 161 von SEQ ID No: 1 von VEGFR-1 eingeschlossen ist, zu erkennen und sich daran zu binden und das Binden von VEGF-A oder PIGF an VEGFR-1 nicht zu verhindern, umfassend:
- eine Komplementarität bestimmende Region 3 der schweren Kette (CDRH3), die die Sequenz YLGDY (aa 118-122 von SEQ ID No: 23) aufweist und
- eine Komplementarität bestimmende Region 2 der schweren Kette (CDRH2), die die Sequenz EIYPRNGNTNYDEKFKG (aa 69-85 von SEQ ID No: 23) aufweist und
- eine Komplementarität bestimmende Region 1 der schweren Kette (CDRHI), die die Sequenz GYIFTNYWMH (aa 45-54 von SEQ ID No: 23) aufweist und
- eine Komplementarität bestimmende Region 3 der leichten Kette (CDRL3), die die Sequenz FQGSHVPYT (aa 113-121 von SEQ ID No: 25) aufweist und
- eine Komplementarität bestimmende Region 2 der leichten Kette (CDRL2), die die Sequenz KVSNRFS (aa 74-80 von SEQ ID No: 25) aufweist und
- eine Komplementarität bestimmende Region 1 der leichten Kette (CDRLI), die die Sequenz RSSQSIVHSNGNTYLE (aa 43-58 von SEQ ID No: 25) aufweist.

2. Antikörper oder synthetisches oder rekombinantes Fragment davon nach Anspruch 1, der/das in der Lage ist, ein Epitop, das in der Sequenz von aa 156 bis aa 161 von SEQ ID No: 1 von VEGFR-1 eingeschlossen ist, zu erkennen und sich daran zu binden und das Binden von VEGF-A oder PIGF an VEGFR-1 nicht zu verhindern.

3. Antikörper oder synthetisches oder rekombinantes Fragment davon nach Anspruch 1 oder 2, **dadurch gekennzeichnet, dass** er/es durch Gebrauchmachen des Peptids der Sequenz TEGRELVIPARVT (SEQ ID No: 2) erhalten wird.

4. Antikörper oder synthetisches oder rekombinantes Fragment davon nach einem der vorhergehenden Ansprüche, umfassend:
- die variable Region der schweren Kette umfassend im Wesentlichen die Aminosäuresequenz: 
QVQLQQPGSELVRPGASVKLSCKASGYIFTNYWVIHWVKQRPGQGLEWIGEI
YPRNGNTNYDEKFKGKATLTIDTSSSTAYMQLSSLTSEDSAVYYCATYLGDY
WGQGTTLTVSS (aa 20-133 von SEQ ID No: 23)
 und/oder
- die variable Region der leichten Kette umfassend im Wesentlichen die Aminosäuresequenz:

DVLMTQTPLSLPVSLGDQASISCRSSQSIVHSNGNTYLEWFLQKPGQSPKLL
IYKVSNRFSGIPDRFSGSGSGTDFTLKISRVEAEDLGVYFCFQGSHVPYTFG
GGTKLEK (aa 20-131 von SEQ ID No: 25).

5. Antikörper oder synthetisches oder rekombinantes Fragment davon nach einem der vorhergehenden Ansprüche, wobei der Antikörper ein monoklonaler, chimärer, humanisierter oder deimmunisierter Antikörper ist.

6. Antikörper oder synthetisches oder rekombinantes Fragment davon nach einem der vorhergehenden Ansprüche zur Verwendung als Medikament, bevorzugt zur Verwendung bei der Behandlung und/oder Prävention von mit Angiogenese verbundenen Pathologien, noch bevorzugter Tumorpathologien, wie z. B. Melanom, Glioblastom, Kolorektalkarzinom, Brustkarzinom, Nierenkarzinom, Eierstockkarzinom, Gebärmutterhalskarzinom, nichtkleinzelligem Lungenkarzinom und/oder Metastase, Retinopathien wie beispielsweise diabetischer Retinopathie, Neovaskularisation bei altersbedingter exsudativer Makuladegeneration, Makulaödem, das durch Okklusion der Netzhautarterie induziert wird, Choroidneovaskularisation, die durch pathologische Kurzsichtigkeit verursacht wird.

7. Nukleinsäuremolekül, das für den Antikörper oder ein synthetisches oder rekombinantes Fragment davon, wie in den Ansprüchen 1-6 definiert, kodiert, bevorzugt eine Nukleotidsequenz umfassend, die im Wesentlichen aus Folgendem besteht: 
CAGGTCCAACTGCAGCAACCTGGGTCTGAACTGGTGAGGCCTGGAGCTTCAGTG
AAGCTGTCCTGCAAGGCTTCTGGCTACATATTCACCAACTACTGGATGCACTGG
GTGAAGCAGAGGCCTGGACAAGGCCTTGAGTGGATTGGAGAAATTTATCCTCGT
AATGGTAATACTAACTACGATGAGAAGTTCAAGGGCAAGGCCACACTGACTATA
GACACATCCTCCAGCACAGCCTACATGCAGCTCAGCAGCCTGACATCTGAGGAC
TCTGCGGTCTATTACTGTGCCACCTACCTAGGGGACTACTGGGGCCAAGGCACC
ACTCTCACAGTCTCCTCA (nt. 58-399 von SEQ ID No: 22)
 und/oder

GATGTTTTGATGACCCAAACTCCACTCTCCCTGCCTGTCAGCCTTGGAGATCAA
GCCTCCATCTCTTGCAGATCTAGTCAGAGTATTGTTCATAGTAATGGAAACACC
TATTTAGAATGGTTCCTGCAGAAACCAGGCCAGTCTCCAAAGCTCCTGATCTAC
AAAGTTTCCAACCGATTTTCTGGGATCCCAGACAGGTTCAGTGGCAGTGGATCA
GGGACAGATTTCACACTCAAGATCAGTAGAGTGGAGGCTGAGGATCTGGGAGTT
TATTTCTGCTTTCAAGGTTCACATGTTCCGTACACGTTCGGAGGGGGGACCAAG
CTGGAAATAAAA (nt. 58-393 von SEQ ID No: 24).

8. Expressionsvektor, der für den Antikörper oder ein synthetisches oder rekombinantes Fragment davon, wie in den Ansprüchen 1-6 definiert, kodiert, der/das bevorzugt die Nukleinsäure, wie in Anspruch 7 definiert, umfasst.

9. Wirtszelle umfassend die Nukleinsäure nach Anspruch 7 oder den Vektor nach Anspruch 8.

10. Pharmazeutische Zusammensetzung umfassend mindestens den Antikörper oder ein synthetisches oder rekombinantes Fragment davon, wie in den Ansprüchen 1-6 definiert, und pharmazeutisch akzeptable Trägerstoffe, wobei die Zusammensetzung bevorzugt der Verwendung durch parenterale Verabreichung, bevorzugt intravenös, dient.

11. Verfahren zum Herstellen des Antikörpers oder eines synthetischen oder rekombinanten Fragments davon, wie in den Ansprüchen 1-6 definiert, umfassend die Schritte des Züchtens der Wirtszelle nach Anspruch 9 und Reinigens des Antikörpers oder eines synthetischen oder rekombinanten Fragments davon, von der Zellkultur.

## Revendications

1. Anticorps ou fragment synthétique ou recombinant de celui-ci capable de reconnaître et se lier à un épitope compris dans la séquence de l'aa. 149 à l'aa. 161 de SEQ ID NO : 1 du VEGFR-1, et de ne pas empêcher la liaison du VEGF-A ou du PIGE au VEGFR-1, comprenant :
- une région de détermination de complémentarité 3 de la chaîne lourde (CDRH3) ayant la séquence YLGDY (aa. 118 à 122 de SEQ ID NO : 23), et
- une région de détermination de complémentarité 2 de la chaîne lourde (CDRH2) ayant la séquence EIYPRNGNTNYDEKFKG (aa. 69 à 85 de SEQ ID NO : 23), et
- une région de détermination de complémentarité 1 de la chaîne lourde (CDRH1) ayant la séquence GYIFTNYWMH (aa. 45 à 54 de SEQ ID NO : 23), et
- une région de détermination de complémentarité 3 de la chaîne légère (CDRL3) ayant la séquence FQGSHVPYT (aa. 113 à 121 de SEQ ID NO : 25), et
- une région de détermination de complémentarité 2 de la chaîne légère (CDRL2) ayant la séquence KVSNRFS (aa. 74 à 80 de SEQ ID NO : 25), et
- une région de détermination de complémentarité 1 de la chaîne légère (CDRL1) ayant la séquence RSSQSIVHSNGNTYLE (aa. 43 à 58 de SEQ ID NO : 25).

2. Anticorps ou fragment synthétique ou recombinant de celui-ci selon la revendication 1 capable de reconnaître et se lier à un épitope compris dans la séquence de l'aa. 156 à l'aa. 161 de SEQ ID NO : 1 du VEGFR-1, et de ne pas empêcher la liaison du VEGF-A ou du PlGF au VEGFR-1.

3. Anticorps ou fragment synthétique ou recombinant de celui-ci selon la revendication 1 ou 2 **caractérisé en ce qu'**il est obtenu en utilisant le peptide de la séquence TEGRELVIPARVT (SEQ ID NO : 2).

4. Anticorps ou fragment synthétique ou recombinant de celui-ci selon l'une quelconque des revendications précédentes comprenant :
- la région variable de la chaîne lourde comprenant essentiellement la séquence d'acides aminés : 
QVQLQQPGSELVRPGASVKLSCKASGYIFTNYWMHWVKQRPGQGLEWIGEIY
PRNGNTNYDEKFKGKATLTIDTSSSTAYMQLSSLTSEDSAVYYCATYLGDYW
GQGTTLTVSS (aa. 20 à 133 de SEQ ID NO : 23)
 et/ou
- la région variable de la chaîne légère comprenant essentiellement la séquence d'acides aminés :

DVLMTQTPLSLPVSLGDQASISCRSSQSIVHSNGNTYLEWFLQKPGQSPKLL
IYKVSNRFSGIPDRFSGSGSGTDFTLKISRVEAEDLGVYFCFQGSHVPYTFG
GGTKLEIK (aa. 20 à 131 de SEQ ID NO : 25).

5. Anticorps ou fragment synthétique ou recombinant de celui-ci selon l'une quelconque des revendications précédentes, dans lequel ledit anticorps est un anticorps monoclonal, chimérique, humanisé ou désimmunisé.

6. Anticorps ou fragment synthétique ou recombinant de celui-ci selon l'une quelconque des revendications précédentes, pour son utilisation en tant que médicament, de préférence pour son utilisation dans le traitement et/ou la prévention de pathologies associées à l'angiogenèse, de manière davantage préférée de pathologies tumorales, par exemple un mélanome, un glioblastome, un carcinome colorectal, un carcinome mammaire, un carcinome rénal, un carcinome ovarien, un carcinome du col de l'utérus et/ou des métastases du poumon non à petites cellules, des rétinopathies par exemple une rétinopathie diabétique, une néovascularisation dans la dégénérescence maculaire humide liée à l'âge, un œdème maculaire induit par l'occlusion de l'artère rétinienne, une néovascularisation choroïdienne provoquée par une myopie pathologique.

7. Molécule d'acide nucléique codant pour l'anticorps ou un fragment synthétique ou recombinant de celui-ci tel que défini dans l'une des revendications 1 à 6, comprenant de préférence une séquence nucléotidique essentiellement constituée de : 
CAGGTCCAACTGCAGCAACCTGGGTCTGAACTGGTGAGGCCTGGAGCTTCAGTG
AAGCTGTCCTGCAAGGCTTCTGGCTACATATTCACCAACTACTGGATGCACTGG
GTGAAGCAGAGGCCTGGACAAGGCCTTGAGTGGATTGGAGAAATTTATCCTCGT
AATGGTAATACTAACTACGATGAGAAGTTCAAGGGCAAGGCCACACTGACTATA
GACACATCCTCCAGCACAGCCTACATGCAGCTCAGCAGCCTGACATCTGAGGAC
TCTGCGGTCTATTACTGTGCCACCTACCTAGGGGACTACTGGGGCCAAGGCACC
ACTCTCACAGTCTCCTCA (nt. 58 à 399 de SEQ ID NO : 22)
 et/ou

GATGTTTTGATGACCCAAACTCCACTCTCCCTGCCTGTCAGCCTTGGAGATCAA
GCCTCCATCTCTTGCAGATCTAGTCAGAGTATTGTTCATAGTAATGGAAACACC
TATTTAGAATGGTTCCTGCAGAAACCAGGCCAGTCTCCAAAGCTCCTGATCTAC
AAAGTTTCCAACCGATTTTCTGGGATCCCAGACAGGTTCAGTGGCAGTGGATCA
GGGACAGATTTCACACTCAAGATCAGTAGAGTGGAGGCTGAGGATCTGGGAGTT
TATTTCTGCTTTCAAGGTTCACATGTTCCGTACACGTTCGGAGGGGGGACCAAG
CTGGAAATAAAA (nt. 58 à 393 de SEQ ID NO : 24).

8. Vecteur d'expression codant pour l'anticorps ou un fragment synthétique ou recombinant de celui-ci tel que défini dans les revendications 1 à 6, de préférence comprenant l'acide nucléique selon la revendication 7.

9. Cellule hôte comprenant l'acide nucléique selon la revendication 7, ou le vecteur selon la revendication 8.

10. Composition pharmaceutique comprenant au moins l'anticorps ou un fragment synthétique ou recombinant de celui-ci tel que défini dans les revendications 1 à 6 et des excipients pharmaceutiquement acceptables, de préférence ladite composition étant destinée à être utilisée par administration parentérale, de préférence intraveineuse.

11. Procédé de production de l'anticorps ou d'un fragment synthétique ou recombinant de celui-ci tel que défini dans les revendications 1 à 6 comprenant les étapes de culture de la cellule hôte selon la revendication 9 et de purification de l'anticorps ou d'un fragment synthétique ou recombinant de celui-ci à partir de la culture cellulaire.
